# EUROPEAN PATENT SPECIFICATION

(11) **EP 2 861 563 B1**
(45) Date of publication and mention of the grant of the patent: **12.07.2017**
(21) Application number: 13726005.5
(22) Date of filing: 31.05.2013
(51) Int. Cl.: C07D 231/56, C07D 235/26, C07D 235/30, A61K 31/4184, A61K 31/4162, A61P 17/06, A61P 19/02, A61P 29/00

(54) **BICYCLIC HETEROCYCLES CAPABLE OF MODULATING T-CELL RESPONSES, AND METHODS OF USING SAME**
BICYCLISCHE HETEROZYKLEN ZUR MODULIERUNG VON T-ZELL-ANTWORTEN UND VERWENDUNGSVERFAHREN DAFÜR
HÉTÉROCYCLES BICYCLIQUES APTES À MODULER DES RÉPONSES DES LYMPHOCYTES T, ET PROCÉDÉS D'UTILISATION ASSOCIÉS

(30) Priority: 01.06.2012 US 201261654398 P
(43) Date of publication of application: 22.04.2015
(73) Proprietor: Nogra Pharma Limited, Dublin 2 (IE)
(72) Inventor: HOMMES, Daan, Los Angeles, CA 90049 (US); VERHAAR, Auke, NL-2801 CB Gouda (NL); VAN DEN BRINK, Gijs, NL-1391 BZ Abcoude (NL); VITI, Francesca, 6872 Salorino (CH)
(74) Representative: Tomkins & Co
(86) International application number: PCT/EP2013/061328
(87) International publication number: WO 2013/178815

(56) References cited:
- WO-A1-2007/024680
- WO-A1-2008/036244
- WO-A2-03/105779
- WO-A2-2007/084728
- HOLLOWAY ET AL: "Discovery of 2-iminobenzimidazoles as a new class of trypanothione reductase inhibitor by high-throughput screening", BIOORGANIC & MEDICINAL CHEMISTRY LETTERS, PERGAMON, ELSEVIER SCIENCE, GB, vol. 17, no. 5, 14 February 2007 (2007-02-14), pages 1422-1427, XP005888492, ISSN: 0960-894X, DOI: 10.1016/J.BMCL.2006.11.090
- HAYES ET AL: "Lead identification of 2-iminobenzimidazole antagonists of the chemokine receptor CXCR3", BIOORGANIC & MEDICINAL CHEMISTRY LETTERS, PERGAMON, ELSEVIER SCIENCE, GB, vol. 18, no. 7, 26 February 2008 (2008-02-26), pages 2414-2419, XP022574973, ISSN: 0960-894X
- VARMA ET AL: "Synthesis of 5-Methoxy-1,3-disubstituted-benzimidazolin -2-thiones as Potential Biologically Active Agents", JOURNAL OF THE INDIAN CHEMICAL SOCIETY, vol. 62, 1985, pages 73-75, XP008163370,

## Description

### BACKGROUND

T lymphocytes (*i.e.,* T cells) are a group of white blood cells that play a major role in cell-mediated immunity. T lymphocytes are activated through the T-Cell Receptor (TCR), a cell type specific receptor. When the TCR is stimulated, another T cell specific protein called lymphocyte-specific protein tyrosine kinase (LCK) is recruited. After recruitment of LCK, a specific signaling cascade is activated, ultimately resulting in the proliferation and production of cytokines (e.g., immunomodulating agents, such as interleukins and interferons).

There are two main subsets of T lymphocytes that are distinguished by the presence of cell surface molecules CD4 and CD8. The T lymphocytes expressing CD4 are regarded as being major cytokine producers. The CD4 subset can be further divided into Th1 and Th2, and the cytokines they produce are known as Th1-type cytokines and Th2-type cytokines.

Chronic immune inflammatory diseases such as rheumatoid arthritis and Crohn's disease are disorders associated with a Th1 response. Th1 cytokines such as interferon gamma tend to produce the pro-inflammatory responses responsible for killing intracellular parasites and for perpetuating autoimmune responses. The response involves recruitment of activated T lymphocytes which infiltrate local tissue causing damage to it. Excessive pro-inflammatory responses can lead to uncontrolled tissue damage, so there needs to be a counteracting mechanism.

The glucocorticoids (GCs) are steroid hormones that act as powerful immunosuppressive drugs, suppressing inflammation on many levels. GCs can passively cross the cellular membrane and bind their cognate receptor, which then moves into the nucleus where it binds DNA and regulates specific genes. Although this is considered to be the main mechanism of action, GC treatment also causes rapid effects on signaling molecules and pathways, for example, by the inhibition of TCR-related signaling. GC treatment causes disruption of a complex of proteins, including the TCR, LCK and the glucocorticoid receptor (GR), and as a consequence downstream signaling is inhibited and proliferation stops. The treatment often results in severe side effects. However, alternative ways of T cell stimulation, which cause activation of alternative signaling pathways, may also lead to proliferation that can not be inhibited by GCs.

WO2007084728 describes 2-imino-benzimidazole compounds. The potential utility of said compounds for a wide variety of diseases is described. No assessment of biological activity is conducted in WO2007084728.

Holloway et al. (Bioorg. Med. Chem. Lett. 2007, 17, 1422) describes a high-throughput screening campaign to assess potential trypanothione reductase inhibition by 2-iminobenzimidazoles.

Hayes et al. (Bioorg. Med. Chem. Lett. 2008, 18, 2414) describes a 2-iminobenzimidazole antagonist of the chemokine receptor CXCR3.

WO03105779 describes guanidine compounds which are described as having potential anesthetic and/or analgesic properties.

WO2007024680 describes pyrazolopyridine and pyrazolopyrimidine compounds with potential use as kinase enzyme modulators.

WO2008036244 describes the use of cyclosporine A to sensitize resistant cancer cells to death receptor ligands.

Varma et al. (J. Indian Chem. Soc. 1985, 62, 73-75) describes the synthesis of several 5-methoxy-1,3-disubstituted benzimidazolin-2-thiones.

There is an unmet need for new therapies to treat immune inflammatory disorders.

### SUMMARY

Provided herein are compounds contemplated to be T-Cell receptor modulators, and their use as, for example, medicinal agents. Accordingly, one aspect of the disclosure provides a compound of Formula IIA: 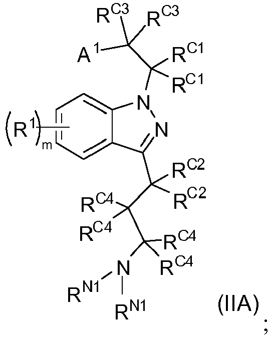 or
a pharmaceutically acceptable salt thereof, wherein the variables are as defined in the detailed description.

Also provided herein are compounds for use in treating a disease or disorder selected from the group consisting of acute and chronic immune inflammatory disorders, e.g., Crohn's disease, ulcerative colitis, rheumatic disorders, psoriasis, and allergies and intestinal cancer, as set out in the claims. A pharmaceutically effective amount of a heterocyclic compound described herein, e.g., a compound of Formula IIA is administered.

### BRIEF DESCRIPTION OF THE DRAWINGS

**Figure 1(A)****,** **(B) and (C)** depict the effect of disclosed compounds on T lymphocyte proliferation.
**Figure 2(A)** depicts the effect of compounds S3.1 and S3.4 on T lymphocyte signal transduction and **(B)** depicts the effect of compounds S3.1 and S3.4 on the interaction between lymphocyte-specific protein tyrosine kinase (LCK) and its chaperone Hsp90.
**Figure 3** depicts the effect of compounds S3.1 and S3.4 on HELA cervical cancer cells in a MTS viability assay.
**Figure 4(A)** and **(B)** depict the effect of compounds S3.1 and S3.4 on differentiation of fibroblasts into adipocytes.
**Figure 5(A)** and **(B)** depict the effect of compounds S3.1 and S3.4 on muscle fiber thickness.
**Figure 6** depicts the NMR spectrum of compound 3.1.

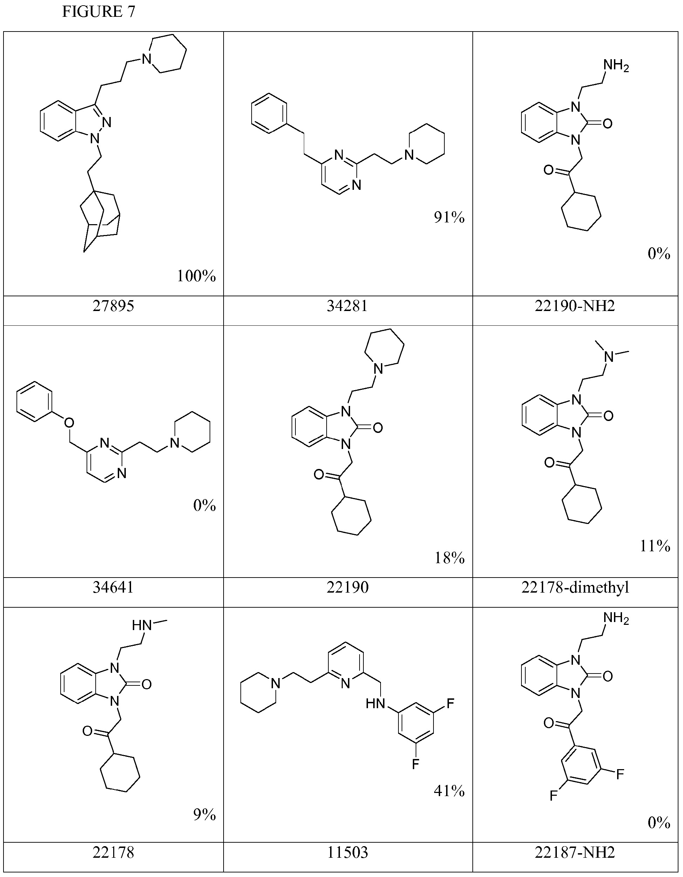

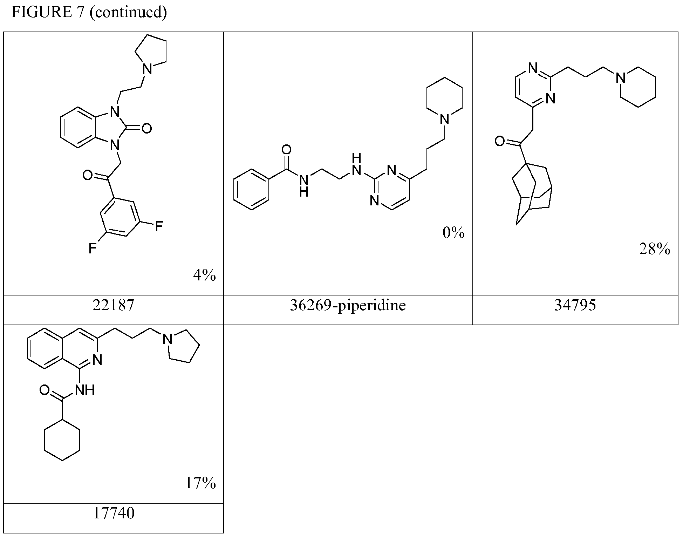

**Figure 7** shows percent inhibition in a lymphocyte proliferation assay for disclosed compounds.
**Figure 8A-8D** depict the NMR spectra of disclosed compounds.

### DETAILED DESCRIPTION

The disclosure is based, in part, upon the discovery that certain compounds disclosed herein have the ability to modulate the activity of T-cell receptors.

Before further description, certain terms employed in the specification, examples and appended claims are collected here. These definitions should be read in light of the remainder of the disclosure and understood as by a person of skill in the art. Unless defined otherwise, all technical and scientific terms used herein have the same meaning as commonly understood by a person of ordinary skill in the art.

"Treating" includes any effect, e.g., lessening, reducing, modulating, or eliminating, that results in the improvement of the condition, disease, disorder and the like.

The term "alkenyl" as used herein refers to an unsaturated straight or branched hydrocarbon having at least one carbon-carbon double bond. Exemplary alkenyl groups include, but are not limited to, a straight or branched group of 2-6 or 3-4 carbon atoms, referred to herein as C₂₋₆alkenyl, and C₃₋₄alkenyl, respectively. Exemplary alkenyl groups include, but are not limited to, vinyl, allyl, butenyl, pentenyl, etc.

The term "alkoxy" as used herein refers to a straight or branched alkyl group attached to oxygen (alkyl-O-). Exemplary alkoxy groups include, but are not limited to, alkoxy groups of 1-6 or 2-6 carbon atoms, referred to herein as C₁₋₆alkoxy, and C₂₋₆alkoxy, respectively. Exemplary alkoxy groups include, but are not limited to methoxy, ethoxy, isopropoxy, etc.

The term "alkoxyalkyl" as used herein refers to a straight or branched alkyl group attached to oxygen, attached to a second straight or branched alkyl group (alkyl-O-alkyl-). Exemplary alkoxyalkyl groups include, but are not limited to, alkoxyalkyl groups in which each of the alkyl groups independently contains 1-6 carbon atoms, referred to herein as C₁₋₆alkoxy-C₁₋₆alkyl. Exemplary alkoxyalkyl groups include, but are not limited to methoxymethyl, 2-methoxyethyl, 1-methoxyethyl, 2-methoxypropyl, ethoxymethyl, 2-isopropoxyethyl etc.

The term "alkyoxycarbonyl" as used herein refers to a straight or branched alkyl group attached to oxygen, attached to a carbonyl group (alkyl-O-C(O)-). Exemplary alkoxycarbonyl groups include, but are not limited to, alkoxycarbonyl groups of 1-6 carbon atoms, referred to herein as C₁₋₆alkoxycarbonyl. Exemplary alkoxycarbonyl groups include, but are not limited to, methoxycarbonyl, ethoxycarbonyl, t-butoxycarbonyl, etc.

The term "alkenyloxy" used herein refers to a straight or branched alkenyl group attached to oxygen (alkenyl-O-). Exemplary alkenyloxy groups include, but are not limited to, groups with an alkenyl group of 3-6 carbon atoms, referred to herein as C₃₋₆alkenyloxy. Exemplary "alkenyloxy" groups include, but are not limited to allyloxy, butenyloxy, etc.

The term "alkynyloxy" used herein refers to a straight or branched alkynyl group attached to oxygen (alkynyl-O). Exemplary alkynyloxy groups include, but are not limited to, groups with an alkynyl group of 3-6 carbon atoms, referred to herein as C₃₋₆alkynyloxy. Exemplary alkynyloxy groups include, but are not limited to, propynyloxy, butynyloxy, etc.

The term "alkyl" as used herein refers to a saturated straight or branched hydrocarbon. Exemplary alkyl groups include, but are not limited to, straight or branched hydrocarbons of 1-6, 1-4, or 1-3 carbon atoms, referred to herein as C₁₋₆alkyl, C₁₋₄alkyl, and C₁₋₃alkyl, respectively. Exemplary alkyl groups include, but are not limited to, methyl, ethyl, propyl, isopropyl, 2-methyl-1-butyl, 3-methyl-2-butyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, butyl, isobutyl, t-butyl, pentyl, isopentyl, neopentyl, hexyl, etc.

The term "alkylcarbonyl" as used herein refers to a straight or branched alkyl group attached to a carbonyl group (alkyl-C(O)-). Exemplary alkylcarbonyl groups include, but are not limited to, alkylcarbonyl groups of 1-6 atoms, referred to herein as C₁₋₆alkylcarbonyl groups. Exemplary alkylcarbonyl groups include, but are not limited to, acetyl, propanoyl, isopropanoyl, butanoyl, etc.

The term "alkynyl" as used herein refers to an unsaturated straight or branched hydrocarbon having at least one carbon-carbon triple bond. Exemplary alkynyl groups include, but are not limited to, straight or branched groups of 2-6, or 3-6 carbon atoms, referred to herein as C₂₋₆alkynyl, and C₃₋₆alkynyl, respectively. Exemplary alkynyl groups include, but are not limited to, ethynyl, propynyl, butynyl, pentynyl, hexynyl, methylpropynyl, etc.

The term "carbonyl" as used herein refers to the group-C(O)-.

The term "cyano" as used herein refers to the group -CN.

The term "cycloalkoxy" as used herein refers to a cycloalkyl group attached to oxygen (cycloalkyl-O-). Exemplary cycloalkoxy groups include, but are not limited to, cycloalkoxy groups of 3-6 carbon atoms, referred to herein as C₃₋₆cycloalkoxy groups. Exemplary cycloalkoxy groups include, but are not limited to, cyclopropoxy, cyclobutoxy, cyclohexyloxy, etc

The terms "cycloalkyl" or a "carbocyclic group" as used herein refers to a saturated or partially unsaturated hydrocarbon group of, for example, 3-6, or 4-6 carbons, referred to herein as C₃₋₆cycloalkyl or C₄₋₆cycloalkyl, respectively. Exemplary cycloalkyl groups include, but are not limited to, cyclohexyl, cyclopentyl, cyclopentenyl, cyclobutyl or cyclopropyl.

The terms "halo" or "halogen" as used herein refer to F, Cl, Br, or I.

The terms "heteroaryl" or "heteroaromatic group" as used herein refers to a monocyclic aromatic 5-6 membered ring system containing one or more heteroatoms, for example one to three heteroatoms, such as nitrogen, oxygen, and sulfur. Where possible, said heteroaryl ring may be linked to the adjacent radical though carbon or nitrogen. Examples of heteroaryl rings include but are not limited to furan, thiophene, pyrrole, thiazole, oxazole, isothiazole, isoxazole, imidazole, pyrazole, triazole, pyridine or pyrimidine etc.

The terms "heterocyclyl" or "heterocyclic group" are art-recognized and refer to saturated or partially unsaturated 4-7 membered ring structures, whose ring structures include one to three heteroatoms, such as nitrogen, oxygen, and sulfur. Where possible, heterocyclyl rings may be linked to the adjacent radical through carbon or nitrogen. Examples of heterocyclyl groups include, but are not limited to, pyrrolidine, piperidine, morpholine, thiomorpholine, piperazine, oxetane, azetidine, tetrahydrofuran or dihydrofuran etc.

The term "heterocyclyloxy" as used herein refers to a heterocyclyl group attached to oxygen (heterocyclyl-O-).

The term "heteroaryloxy" as used herein refers to a heteroaryl group attached to oxygen (heteroaryl-O-).

The terms "hydroxy" and "hydroxyl" as used herein refers to the group -OH.

The term "oxo" as used herein refers to the group =O.

"Pharmaceutically or pharmacologically acceptable" include molecular entities and compositions that do not produce an adverse, allergic or other untoward reaction when administered to an animal, or a human, as appropriate. For human administration, preparations should meet sterility, pyrogenicity, and general safety and purity standards as required by FDA Office of Biologics standards.

The term "pharmaceutically acceptable carrier" or "pharmaceutically acceptable excipient" as used herein refers to any and all solvents, dispersion media, coatings, isotonic and absorption delaying agents, and the like, that are compatible with pharmaceutical administration. The use of such media and agents for pharmaceutically active substances is well known in the art. The compositions may also contain other active compounds providing supplemental, additional, or enhanced therapeutic functions.

The term "pharmaceutical composition" as used herein refers to a composition comprising at least one compound as disclosed herein formulated together with one or more pharmaceutically acceptable carriers.

"Individual," "patient," or "subject" are used interchangeably and include any animal, including mammals, e.g., mice, rats, other rodents, rabbits, dogs, cats, swine, cattle, sheep, horses, or primates, and humans. In an embodiment, the patient is a human. The compounds contemplated herein can be administered to a mammal, such as a human, but can also be administered to other mammals such as an animal in need of veterinary treatment, e.g., domestic animals (e.g., dogs, cats, and the like), farm animals (e.g., cows, sheep, pigs, horses, and the like) and laboratory animals (e.g., rats, mice, guinea pigs, and the like). The mammal treated in the methods contemplated herein is desirably a mammal in which treatment of obesity or weight loss is desired. "Modulation" includes antagonism (e.g., inhibition), agonism, partial antagonism and/or partial agonism.

In the present specification, the term "therapeutically effective amount" means the amount of the subject compound that will elicit the biological or medical response of a tissue, system or animal (e.g., mammal or human) that is being sought by the researcher, veterinarian, medical doctor or other clinician. The compounds contemplated herein are administered in therapeutically effective amounts to treat a disease or disorder. Alternatively, a therapeutically effective amount of a compound is the quantity required to achieve a desired therapeutic and/or prophylactic effect, such as an amount, which results in weight loss.

The term "pharmaceutically acceptable salt(s)" as used herein refers to salts of acidic or basic groups that may be present in compounds used in the compositions. Compounds included in the present compositions that are basic in nature are capable of forming a wide variety of salts with various inorganic and organic acids. The acids that may be used to prepare pharmaceutically acceptable acid addition salts of such basic compounds are those that form non-toxic acid addition salts, i.e., salts containing pharmacologically acceptable anions, including, but not limited to, malate, oxalate, chloride, bromide, iodide, nitrate, sulfate, bisulfate, phosphate, acid phosphate, isonicotinate, acetate, lactate, salicylate, citrate, tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate and pamoate (i.e., 1,1'-methylene-bis-(2-hydroxy-3-naphthoate)) salts. Compounds included in the present compositions that are acidic in nature are capable of forming base salts with various pharmacologically acceptable cations. Examples of such salts include alkali metal or alkaline earth metal salts and, particularly, calcium, magnesium, sodium, lithium, zinc, potassium, and iron salts. Compounds included in the present compositions that include a basic or acidic moiety may also form pharmaceutically acceptable salts with various amino acids. The compounds of the disclosure may contain both acidic and basic groups; for example, one amino and one carboxylic acid group. In such a case, the compound can exist as an acid addition salt, a zwitterion, or a base salt.

The compounds of the disclosure may contain one or more chiral centers and, therefore, exist as stereoisomers. The term "stereoisomers" when used herein consist of all enantiomers or diastereomers. These compounds may be designated by the symbols "(+)," "(-)," "R" or "S," depending on the configuration of substituents around the stereogenic carbon atom, but the skilled artisan will recognize that a structure may denote a chiral center implicitly. The present disclosure encompasses various stereoisomers of these compounds and mixtures thereof. Mixtures of enantiomers or diastereomers may be designated "(±)" in nomenclature, but the skilled artisan will recognize that a structure may denote a chiral center implicitly.

Individual enantiomers and diastereomers of compounds of the present disclosure can be prepared synthetically from commercially available starting materials that contain asymmetric or stereogenic centers, or by preparation of racemic mixtures followed by resolution methods well known to those of ordinary skill in the art. These methods of resolution are exemplified by (1) attachment of a mixture of enantiomers to a chiral auxiliary, separation of the resulting mixture of diastereomers by recrystallization or chromatography and liberation of the optically pure product from the auxiliary, (2) salt formation employing an optically active resolving agent, (3) direct separation of the mixture of optical enantiomers on chiral liquid chromatographic columns or (4) kinetic resolution using stereoselective chemical or enzymatic reagents. Racemic mixtures can also be resolved into their component enantiomers by well known methods, such as chiral-phase liquid chromatography or crystallizing the compound in a chiral solvent. Stereoselective syntheses, a chemical or enzymatic reaction in which a single reactant forms an unequal mixture of stereoisomers during the creation of a new stereocenter or during the transformation of a pre-existing one, are well known in the art. Stereoselective syntheses encompass both enantio- and diastereoselective transformations, and may involve the use of chiral auxiliaries. For examples, see Carreira and Kvaerno, Classics in Stereoselective Synthesis, Wiley-VCH: Weinheim, 2009.

The compounds disclosed herein can exist in solvated as well as unsolvated forms with pharmaceutically acceptable solvents such as water, ethanol, and the like, and it is intended that the disclosure embrace both solvated and unsolvated forms. In one embodiment, the compound is amorphous. In one embodiment, the compound is a single polymorph. In another embodiment, the compound is a mixture of polymorphs. In another embodiment, the compound is in a crystalline form.

The disclosure also embraces isotopically labeled compounds of the disclosure which are identical to those recited herein, except that one or more atoms are replaced by an atom having an atomic mass or mass number different from the atomic mass or mass number usually found in nature. Examples of isotopes that can be incorporated into compounds of the disclosure include isotopes of hydrogen, carbon, nitrogen, oxygen, phosphorus, sulfur, fluorine and chlorine, such as ²H, ³H, ¹³C, ¹⁴C, ¹⁵N, ¹⁸O, ¹⁷O, ³¹P, ³²P, ³⁵S, ¹⁸F, and ³⁶Cl, respectively. For example, a compound of the disclosure may have one or more H atom replaced with deuterium.

Certain isotopically-labeled disclosed compounds (e.g., those labeled with ³H and ¹⁴C) are useful in compound and/or substrate tissue distribution assays. Tritiated (i.e., ³H) and carbon-14 (i.e., ¹⁴C) isotopes may be useful for their ease of preparation and detectability. Further, substitution with heavier isotopes such as deuterium (i.e., ²H) may afford certain therapeutic advantages resulting from greater metabolic stability (e.g., increased *in vivo* half-life or reduced dosage requirements) and hence may be preferred in some circumstances. Isotopically labeled compounds contemplated herein can generally be prepared by following procedures analogous to those disclosed in the examples herein by substituting an isotopically labeled reagent for a non-isotopically labeled reagent.

The term "prodrug" refers to compounds that are transformed *in vivo* to yield a disclosed compound or a pharmaceutically acceptable salt, hydrate or solvate of the compound. The transformation may occur by various mechanisms (such as by esterase, amidase, phosphatase, oxidative and or reductive metabolism) in various locations (such as in the intestinal lumen or upon transit of the intestine, blood or liver). In one embodiment, the transformation may be by, for example, chemical hydrolysis or enzymatic attack. Prodrugs are well known in the art (for example, see Rautio, Kumpulainen, et al., Nature Reviews Drug Discovery 2008, 7, 255). For example, if a compound contemplated herein or a pharmaceutically acceptable salt, hydrate or solvate of the compound contains a carboxylic acid functional group, a prodrug can comprise an ester formed by the replacement of the hydrogen atom of the acid group with a group such as (C₁₋₈)alkyl, (C₂₋₁₂)alkanoyloxymethyl, 1-(alkanoyloxy)ethyl having from 4 to 9 carbon atoms, 1-methyl-1-(alkanoyloxy)-ethyl having from 5 to 10 carbon atoms, alkoxycarbonyloxymethyl having from 3 to 6 carbon atoms, 1-(alkoxycarbonyloxy)ethyl having from 4 to 7 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N-(alkoxycarbonyl)amino)ethyl having from 4 to 10 carbon atoms, 3-phthalidyl, 4-crotonolactonyl, gamma-butyrolacton-4-yl, di-N,N-(C₁₋₂)alkylamino(C₂₋₃)alkyl (such as β-dimethylaminoethyl), carbamoyl-(C₁₋₂)alkyl, N,N-di(C₁₋₂)alkylcarbamoyl-(C₁₋₂)alkyl and piperidino-, pyrrolidino- or morpholino(C₂₋₃)alkyl.

Similarly, if a compound contemplated herein contains an alcohol functional group, a prodrug can be formed by the replacement of the hydrogen atom of the alcohol group with a group such as (C₁₋₆)alkanoyloxymethyl, 1-((C₁₋₆)alkanoyloxy)ethyl, 1-methyl-1-((C₁₋₆)alkanoyloxy)ethyl (C₁₋₆)alkoxycarbonyloxymethyl, N-(C1-6)alkoxycarbonylaminomethyl, succinoyl, (C₁₋₆)alkanoyl, α-amino(C₁₋₄)alkanoyl, arylacyl and α-aminoacyl, or α-aminoacyl-α-aminoacyl, where each α-aminoacyl group is independently selected from the naturally occurring L-amino acids, -P(O)(OH)₂, -P(O)(O(C₁₋₆)alkyl)₂ or glycosyl (the radical resulting from the removal of a hydroxyl group of the hemiacetal form of a carbohydrate).

If a compound contemplated herein incorporates an amine functional group, a prodrug can be formed, for example, by creation of an amide or carbamate, an N-acyloxyakyl derivative, an (oxodioxolenyl)methyl derivative, an N-Mannich base, imine or enamine. In addition, a secondary amine can be metabolically cleaved to generate a bioactive primary amine, or a tertiary amine can metabolically cleaved to generate a bioactive primary or secondary amine. For examples, see Simplício, et al., Molecules 2008, 13, 519 and references therein.

A derivative or pro-drug can have enhanced permeability for a target organ. The prodrug has an enhanced permeability according to the present disclosure if, after administration of the pro-drug or derivative thereof to a living organism, a higher amount of the compound reaches the target organ, resulting in a higher level of effective agent, as compared to administration of the base compound without derivatization.

Further, if a variable is not accompanied by a definition, then the previous definition of the variable controls.

### T Cell Receptor Ligands

In one aspect, there is provided heterocyclic compounds as set out in the claims.

Heterocyclic compounds of Formula I or Formula II are described herein: 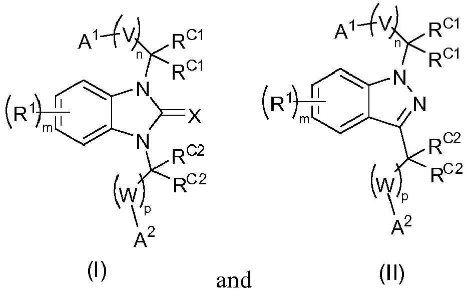 wherein
A¹ is selected from the group consisting of C₃₋₆cycloalkyl, heterocyclyl, phenyl, naphthyl, heteroaryl, C₁₋₆alkyl, hydroxy, -NR'R', -O-C₁₋₆alkyl, -S-C₁₋₆alkyl, -U-C₃₋₆cycloalkyl, -U-heterocyclyl, -U-phenyl, -U-naphthyl, -U-heteroaryl, phenylalkyl, naphthylalkyl, heterocycloalkyl, and heteroarylalkyl; wherein C₃₋₆cycloalkyl, heterocyclyl, phenyl, naphthyl, heteroaryl, and C₁₋₆alkyl are optionally substituted with one, two, three or more substituents each selected from R^{A1};
A² is selected from the group consisting of C₃₋₆cycloalkyl, heterocyclyl, phenyl, naphthyl, heteroaryl, -NR'R', U-C₃₋₆cycloalkyl, -U-heterocyclyl, -U-phenyl, -U-naphthyl, -U-heteroaryl, phenylalkyl, naphthylalkyl, heterocycloalkyl, and heteroarylalkyl; wherein C₃₋₆cycloalkyl, heterocyclyl, phenyl, naphthyl, and heteroaryl are optionally substituted with one, two, three or more substituents each selected from R^{A2};
U is selected from the group consisting of -S-, -NR'-, -O-, or C₁₋₆alkyl;
X is selected from the group consisting of O, S and NR';
V is independently selected, for each occurrence, from the group consisting of O, S, NR', and CR^{C3}R^{C3};
W is independently selected, for each occurrence, from the group consisting of O, S, NR', and CR^{C4}R^{C4};
R^{C1}, R^{C2}, R^{C3} and R^{C4} are independently selected, for each occurrence, from the group consisting of hydrogen, halogen, hydroxyl, amino, amido, sulfonyl, sulfonamide, thiol, C₁₋₆alkyl, haloC₁₋₆alkyl, carboxyl, cyano, nitro, alkoxy, C₃₋₆cycloalkyl, heterocyclyl, phenyl, naphthyl, and heteroaryl;
   or two R^{C3} substituents or two R^{C4} substituents may be taken together to form an oxo, imino or sulfanylidene;
   or two R^{C1} substituents, two R^{C2} substituents, two R^{C3} substituents or two R^{C4} substituents may be taken together with the atom or atoms to which they are attached to form a C₃₋₆cycloalkyl, phenyl, naphthyl, or a saturated, partially saturated or unsaturated, 4-6 membered monocyclic or 10-12 membered bicyclic heterocycle having one, two or three heteroatoms independently selected from the group consisting of N, O, and S;
   or one R^{C1} substituent and one R^{C3} substituent or one R^{C2} substituent and one R^{C4} substituent may be taken together with the atoms to which they are attached to form a C₃₋₆cycloalkyl, phenyl, naphthyl, or a saturated, partially saturated or unsaturated, 4-6 membered monocyclic or 10-12 membered bicyclic heterocycle having one, two or three heteroatoms independently selected from the group consisting ofN, O, and S;
R¹ is independently selected, for each occurrence, from the group consisting of halogen, hydroxyl, amino, amido, sulfonyl, sulfonamide, thiol, C₁₋₆alkyl, haloC₁₋₆alkyl, carboxyl, -C(O)O-C₁₋₆alkyl, -OC(O)O-C₁₋₆alkyl, -OC(O)NR'R', -N(R')C(O)NR'R', -N(R')C(O)O-C₁₋₆alkyl, cyano, nitro, alkoxy, C₃₋₆cycloalkyl, heterocyclyl, heteroaryl and phenyl;
R^{A1} is independently selected, for each occurrence, from the group consisting of halogen, hydroxyl, amino, amido, sulfonyl, sulfonamide, thiol, C₁₋₆alkyl, haloC₁₋₆alkyl, carboxyl, cyano, nitro, alkoxy, -C(O)O-C₁₋₆alkyl, -OC(O)O-C₁₋₆alkyl, -OC(O)NR'R', -N(R')C(O)NR'R', -N(R')C(O)O-C₁₋₆alkyl, oxo, C₃₋₆cycloalkyl, heterocyclyl, heteroaryl and phenyl;
R^{A2} is independently selected, for each occurrence, from the group consisting of halogen, hydroxyl, amino, amido, sulfonyl, sulfonamide, thiol, C₁₋₆alkyl, haloC₁₋₆alkyl, carboxyl, cyano, nitro, alkoxy, -C(O)O-C₁₋₆alkyl, -OC(O)O-C₁₋₆alkyl, -OC(O)NR'R', -N(R')C(O)NR'R', -N(R')C(O)O-C₁₋₆alkyl, oxo, C₃₋₆cycloalkyl, heterocyclyl, heteroaryl and phenyl;
R' is independently selected, for each occurrence, from the group consisting of hydrogen or C₁₋₆alkyl; wherein C₁₋₆alkyl is optionally substituted with one, two, three or more substituents each selected from R^{A1};
   or two R' may be taken together with the atom or atoms to which they are attached to form a saturated, partially saturated or unsaturated, 4-7 membered monocyclic or 10-15 membered bicyclic heterocycle having one, two or three heteroatoms independently selected from the group consisting of N, O, and S;
n is selected from the group consisting of 0, 1, 2, 3 and 4;
p is selected from the group consisting of 0, 1, 2, 3 and 4; and
m is selected from the group consisting of 0, 1, 2 and 3; or
a pharmaceutically acceptable salt or a stereoisomer thereof.

A¹ may be selected from the group consisting of C₃₋₆cycloalkyl and heterocyclyl. For example, A¹ may be C₃₋₆cycloalkyl.

A² may be selected from the group consisting of C₃₋₆cycloalkyl, heterocyclyl, phenyl, naphthyl, heteroaryl, and -NR'R'. For example, A² may be selected from the group consisting of heterocyclyl and heteroaryl.

X may be NH.

V may be selected from the group consisting of C(O) and CH₂.

W may be CH₂.

R^{C1} and R^{C2} may be hydrogen.

n may be selected from the group consisting of 1 and 2.

p may be selected from the group consisting of 1 and 2.

A heterocyclic compound of Formula IA or IB is also described herein: 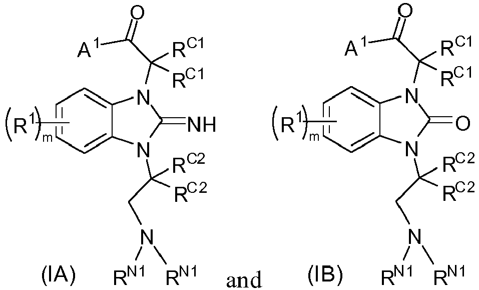 wherein
A¹ is selected from the group consisting of C₃₋₆cycloalkyl, heterocyclyl, phenyl, naphthyl, heteroaryl, C₁₋₆alkyl, hydroxy, -NR'R', -O-C₁₋₆alkyl, -S-C₁₋₆alkyl U-C₃₋₆cycloalkyl, -U-heterocyclyl, -U-phenyl, -U-naphthyl, -U-heteroaryl, phenylalkyl, naphthylalkyl, heterocycloalkyl, and heteroarylalkyl; wherein C₃₋₆cycloalkyl, heterocyclyl, phenyl, naphthyl, heteroaryl, and C₁₋₆alkyl are optionally substituted with one, two, three or more substituents each selected from R^{A1};
U is selected from the group consisting of -S-, -NR'-, -O-, or C₁₋₆alkyl;
R^{C1} and R^{C2} are independently selected, for each occurrence, from the group consisting of hydrogen, halogen, hydroxyl, amino, amido, sulfonyl, sulfonamide, thiol, C₁₋₆alkyl, haloC₁₋₆alkyl, carboxyl, cyano, nitro, alkoxy, C₃₋₆cycloalkyl, heterocyclyl, phenyl, naphthyl, and heteroaryl;
or two R^{C1} substituents or two R^{C2} substituents may be taken together with the atom to which they are attached to form a C₃₋₆cycloalkyl, phenyl, naphthyl, or a saturated, partially saturated or unsaturated, 4-6 membered monocyclic or 10-12 membered bicyclic heterocycle having one, two or three heteroatoms independently selected from the group consisting ofN, O, and S;
R¹ is independently selected, for each occurrence, from the group consisting of halogen, hydroxyl, amino, amido, sulfonyl, sulfonamide, thiol, C₁₋₆alkyl, haloC₁₋₆alkyl, carboxyl, -C(O)O-C₁₋₆alkyl, -OC(O)O-C₁₋₆alkyl, -OC(O)NR'R', -N(R')C(O)NR'R', -N(R')C(O)O-C₁₋₆alkyl, cyano, nitro, alkoxy, C₃₋₆cycloalkyl, heterocyclyl, heteroaryl and phenyl;
R^{A1} is independently selected, for each occurrence, from the group consisting of halogen, hydroxyl, amino, amido, sulfonyl, sulfonamide, thiol, C₁₋₆alkyl, haloC₁₋₆alkyl, carboxyl, cyano, nitro, alkoxy, -C(O)O-C₁₋₆alkyl, -OC(O)O-C₁₋₆alkyl, -OC(O)NR'R', -N(R')C(O)NR'R', -N(R')C(O)O-C₁₋₆alkyl, oxo, C₃₋₆cycloalkyl, heterocyclyl, heteroaryl and phenyl;
R^{N1} and R' are independently selected, for each occurrence, from the group consisting of hydrogen and C₁₋₆alkyl; wherein C₁₋₆alkyl is optionally substituted with one, two, three or more substituents independently selected, for each occurrence, from R^{A1};
   or two R^{N1} substituents or two R' substituents may be taken together with the nitrogen to which they are attached to form a saturated, partially saturated or unsaturated, 4-7 membered monocyclic or 10-15 membered bicyclic heterocycle having one, two or three heteroatoms independently selected from the group consisting of N, O, and S; and m is selected from the group consisting of 0, 1, 2 and 3; or
a pharmaceutically acceptable salt or a stereoisomer thereof.

A¹ may be C₃₋₆cycloalkyl. For example, A¹ may be selected from the group consisting of cyclohexyl, cyclohexenyl, cyclopentyl, cyclopentenyl, cyclobutyl, cyclopropyl, and any bridged, spiro or fused variant thereof.

R^{C1} and R^{C2} may be hydrogen.

In other compounds, 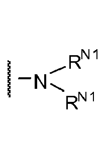 may be selected from the group consisting of: 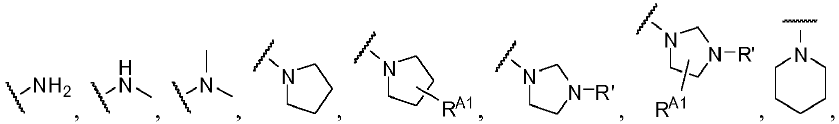 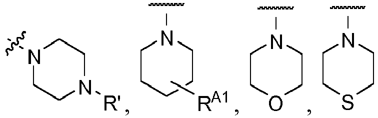 and any pharmaceutically acceptable salts thereof.

In one aspect, a heterocyclic compound of Formula IIA is provided: 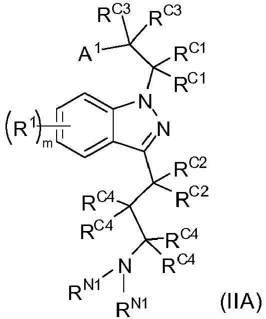 wherein
A¹ is selected from the group consisting of C₃₋₆cycloalkyl, heterocyclyl, phenyl, naphthyl, heteroaryl, C₁₋₆alkyl, hydroxy, -NR'R', -O-C₁₋₆alkyl, -S-C₁₋₆alkyl, - U-C₃₋₆cycloalkyl, -U-heterocyclyl, -U-phenyl, -U-naphthyl, -U-heteroaryl, phenylalkyl, naphthylalkyl, heterocycloalkyl, and heteroarylalkyl; wherein C₃₋₆cycloalkyl, heterocyclyl, phenyl, naphthyl, heteroaryl, and C₁₋₆alkyl are optionally substituted with one, two, three or more substituents each selected from R^{A1};
U is selected from the group consisting of -S-, -NR'-, -O-, or C₁₋₆alkyl;
R^{C1}, R^{C2}, R^{C3} and R^{C4} are independently selected, for each occurrence, from the group consisting of hydrogen, halogen, hydroxyl, amino, amido, sulfonyl, sulfonamide, thiol, C₁₋₆alkyl, haloC₁₋₆alkyl, carboxyl, cyano, nitro, alkoxy, C₃₋₆cycloalkyl, heterocyclyl, phenyl, naphthyl, and heteroaryl;
or two R^{C3} substituents or two R^{C4} substituents may be taken together to form an oxo, imino or sulfanylidene;
or two R^{C1} substituents, two R^{C2} substituents, two R^{C3} substituents or two R^{C4} substituents may be taken together with the atom or atoms to which they are attached to form a C₃₋₆cycloalkyl, phenyl, naphthyl, or a saturated, partially saturated or unsaturated, 4-6 membered monocyclic or 10-12 membered bicyclic heterocycle having one, two or three heteroatoms independently selected from the group consisting of N, O, and S;
or one R^{C1} substituent and one R^{C3} substituent or one R^{C2} substituent and one R^{C4} substituent may be taken together with the atoms to which they are attached to form a C₃₋₆cycloalkyl, phenyl, naphthyl, or a saturated, partially saturated or unsaturated, 4-6 membered monocyclic or 10-12 membered bicyclic heterocycle having one, two or three heteroatoms independently selected from the group consisting ofN, O, and S;
R¹ is independently selected, for each occurrence, from the group consisting of halogen, hydroxyl, amino, amido, sulfonyl, sulfonamide, thiol, C₁₋₆alkyl, haloC₁₋₆alkyl, carboxyl, -C(O)O-C₁₋₆alkyl, -OC(O)O-C₁₋₆alkyl, -OC(O)NR'R', -N(R')C(O)NR'R', -N(R')C(O)O-C₁₋₆alkyl, cyano, nitro, alkoxy, C₃₋₆cycloalkyl, heterocyclyl, heteroaryl and phenyl;
R^{A1} is independently selected, for each occurrence, from the group consisting of halogen, hydroxyl, amino, amido, sulfonyl, sulfonamide, thiol, C₁₋₆alkyl, haloC₁₋₆alkyl, carboxyl, cyano, nitro, alkoxy, -C(O)O-C₁₋₆alkyl, -OC(O)O-C₁₋₆alkyl, -OC(O)NR'R', -N(R')C(O)NR'R', -N(R')C(O)O-C₁₋₆alkyl, oxo, C₃₋₆cycloalkyl, heterocyclyl, heteroaryl and phenyl;
R^{N1} and R' are independently selected, for each occurrence, from the group consisting of hydrogen and C₁₋₆alkyl; wherein C₁₋₆alkyl is optionally substituted with one, two, three or more substituents independently selected, for each occurrence, from R^{A1};
   or two R^{N1} substituents or two R' substituents may be taken together with the nitrogen to which they are attached to form a saturated, partially saturated or unsaturated, 4-7 membered monocyclic or 10-15 membered bicyclic heterocycle having one, two or three heteroatoms independently selected from the group consisting of N, O, and S;
   or one R^{N1} substituent and one R^{C4} substituent may be taken together with the atoms to which they are attached to form a saturated, partially saturated or unsaturated, 4-7 membered monocyclic or 10-15 membered bicyclic heterocycle having one, two or three heteroatoms independently selected from the group consisting of N, O, and S; and m is selected from the group consisting of 0, 1, 2 and 3; or
a pharmaceutically acceptable salt or a stereoisomer thereof

In one embodiment, A¹ may be C₃₋₆cycloalkyl. For example, A¹ may be selected from the group consisting of cyclohexyl, cyclohexenyl, cyclopentyl, cyclopentenyl, cyclobutyl, cyclopropyl, and any bridged, spiro or fused variant thereof

In another embodiment, R^{C1} and R^{C2} may be hydrogen.

In certain embodiments, R^{C3} and R^{C4} may be hydrogen; or two R^{C3} substituents or two R^{C4} substituents are taken together to form oxo.

In other embodiments, 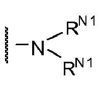 may be selected from the group consisting of: 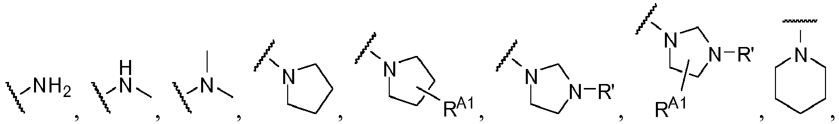 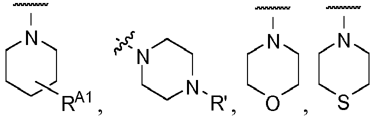 and any pharmaceutically acceptable salts thereof.

In a further aspect, a heterocyclic compound is provided, wherein the heterocyclic compound is selected from the group consisting of: 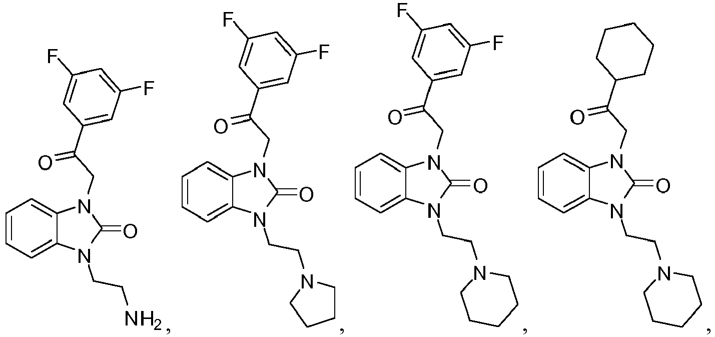 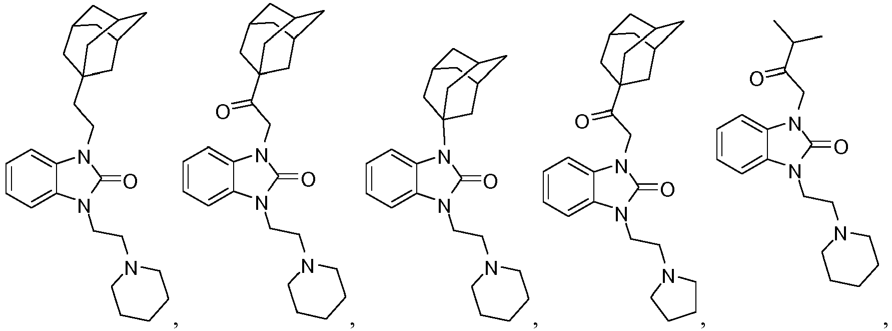 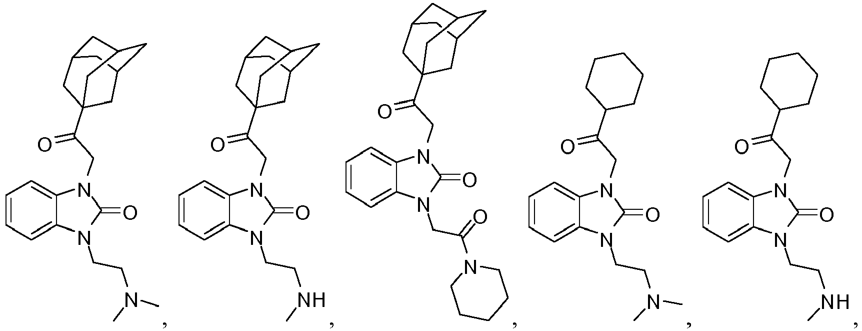 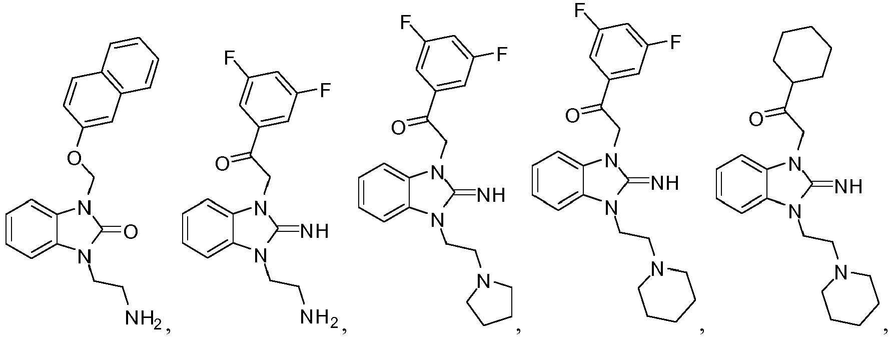 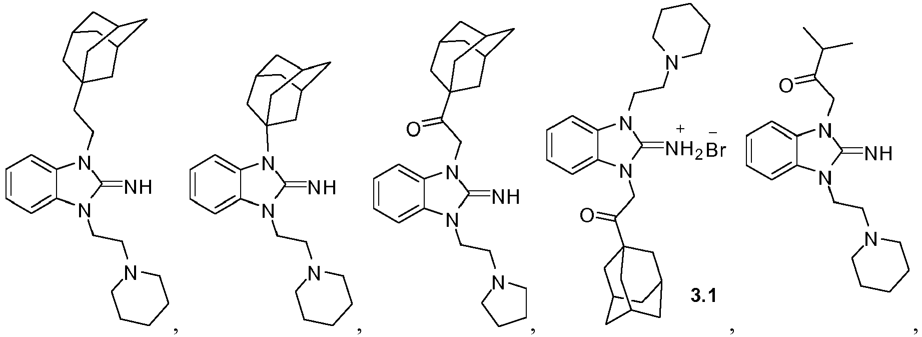 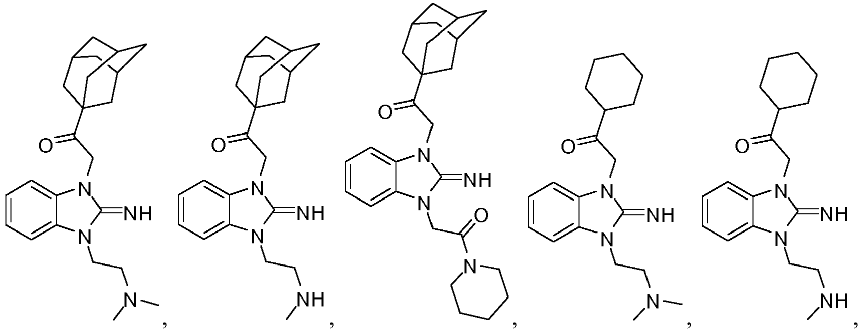 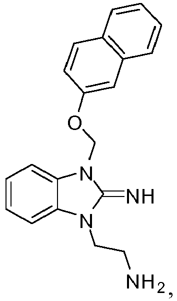 and any pharmaceutically acceptable salts thereof.

In another aspect, a heterocyclic compound is provided, wherein the heterocyclic compound is selected from the group consisting of: 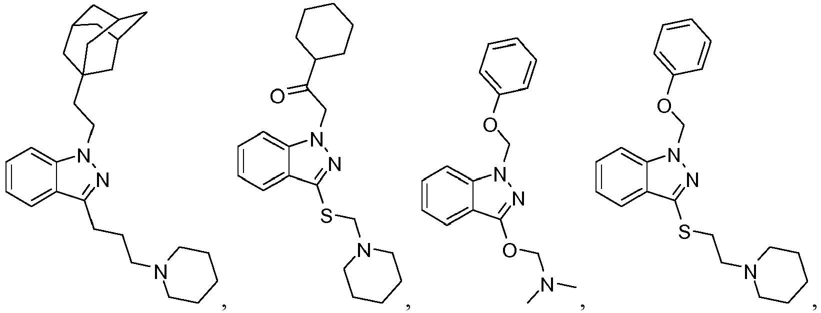 and 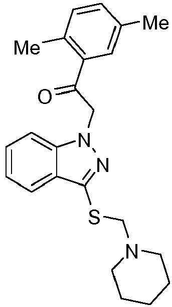 or pharmaceutically acceptable salts thereof.

Procedures for making compounds described herein are provided below. In the reactions described below, it may be necessary to protect reactive functional groups (such as hydroxyl, amino, thio or carboxyl groups) to avoid their unwanted participation in the reactions. The incorporation of such groups, and the methods required to introduce and remove them are known to those skilled in the art (for example, see Greene, Wuts, Protective Groups in Organic Synthesis. 2nd Ed. (1999)). The deprotection step may be the final step in the synthesis such that the removal of protecting groups affords compounds of Formula I, as disclosed herein. Starting materials used in the following schemes can be purchased or prepared by methods described in the chemical literature, or by adaptations thereof, using methods known by those skilled in the art. The order in which the steps are performed can vary depending on the groups introduced and the reagents used, but would be apparent to those skilled in the art.

Multiple methods for preparing compounds described herein are provided in the examples. Further synthetic methods for preparing various compounds described herein are provided by the following schemes. The schemes are given for the purpose of illustrating the invention, but not for limiting the scope or spirit of the invention. Starting materials shown in the schemes can be obtained from commercial sources or can be prepared based on procedures described in the literature.

The synthetic route in Scheme 1 illustrates a general method for preparing benzimidazolone derivatives. The method involves attaching the desired substituents to the nitrogens of the benzimidazolone core. The desired alkyl group attached at the 1-position of the benzimidazolone can be installed by reacting benzimidazolone **A** with the α-bromocarbonyl substrate to provide monosubstituted **B**. The desired functional group at the 3-position of the benzimidazolone can be installed by reacting **B** with the appropriate electrophile (e.g., R-C1) to provide final product **C.**

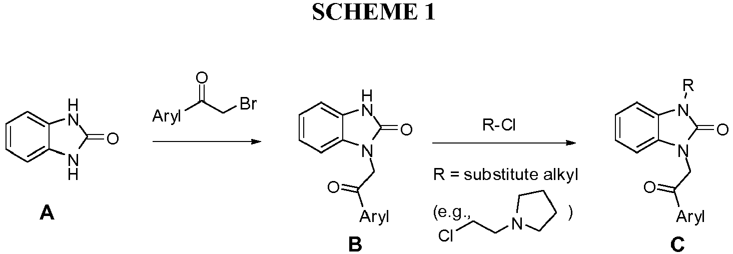

Scheme 2 provides a more detailed exemplary synthetic procedure for making benzimidazolone derivatives having various functional groups (e.g., alkylamino) at the 3-position of the benzimidazolone ring. Aryl **A** can be converted to aniline **B** using boc-protected ethylene diamine followed by reduction of the nitro group. The benzimidazolone core can be installed by reaction with carbonyldiimidazole (CDI). Substitution at the 1-position can be installed by reaction with the appropriate α-bromocarbonyl to afford compound **C**. Deprotection of the boc-protected amino group followed by reaction with the appropriate electrophile affords the final disubstituted compound **D**.

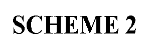

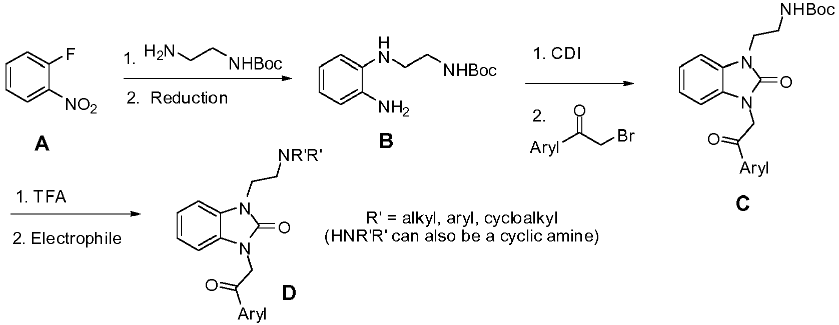

The synthetic route in Scheme 3 illustrates a general method for preparing heterobicyclic derivatives. The method involves attaching the desired substituents to the heterocyclic core. The desired alkyl group attached at the nitrogen of heterocycle **A** can be installed by reacting **A** with the appropriate electrophile to provide monosubstituted **B**. The alkyne group at the 3-position of the heterocycle core can be installed by reacting **B** under Sonogashira reaction conditions to provide alkyne **C**. Reduction of the alkyne provides the dialkyl substituted final product **D**.

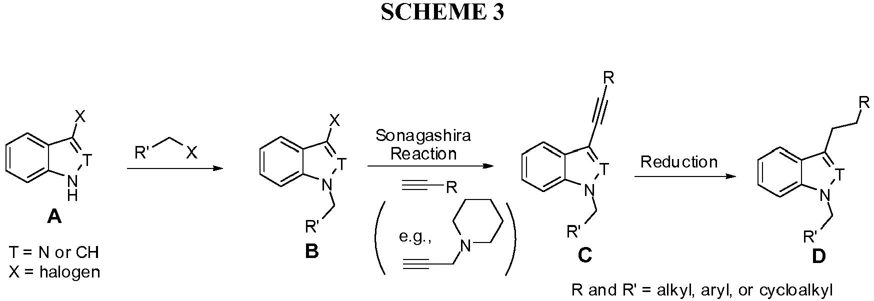

### Methods

One aspect of the disclosure provides methods of modulating the activity of T cell receptors, comprising administering a pharmaceutically effective amount of a disclosed heterocyclic compound to a patient in need thereof. In some embodiments of the invention, the compound utilized by one or more of the foregoing methods is one of the generic, subgeneric, or specific compounds described herein, as a compound of Formula IIA. The ability of compounds described herein to modulate or inhibit T cell receptors can be evaluated by procedures known in the art and/or described herein. Another aspect of the disclosure provides compounds for use in treating a disease associated with expression or activity of T cell receptor. A pharmaceutically effective amount of a disclosed heterocyclic compound, of Formula IIA is administered to a patient in need thereof.

In certain embodiments, the invention provides compounds for use in treating a disease or disorder selected from the group of acute and chronic immune inflammatory disorders, e.g., Crohn's disease, ulcerative colitis, rheumatic disorders, psoriasis, and allergies, as set out in the claims. A pharmaceutically effective amount of a disclosed heterocyclic compound, of Formula IIA, is administered to a patient in need thereof.

Compounds for use in treating gastrointestinal diseases are provided as set out in the claims. A pharmaceutically effective amount of a disclosed heterocyclic compound, e.g. a compound of Formula IIA, is administered to a patient in need thereof. Contemplated methods include, for example, methods of treating inflammatory bowel diseases, e.g., ulcerative colitis, Crohn's disease, indeterminate colitis; and diverticulitis.

Also disclosed herein are compounds for use in treating endocrine disorders. A pharmaceutically effective amount of a disclosed heterocyclic compound, e.g. a compound of Formula I, IA, IB, II, or IIA, is administered to a patient in need thereof. For example, methods of treating congenital adrenal hyperplasia; hypercalciemia associated with cancer; and nonsuppurative thyroiditis are provided.

Compounds for use in treating rheumatic disorders are disclosed. A pharmaceutically effective amount of a disclosed heterocyclic compound, e.g. a compound of Formula I, IA, IB, II, or IIA, is administered to a patient in need thereof. For example, compounds for use in treating psoriatic arthritis; rheumatoid arthritis, including juvenile rheumatoid arthritis; ankylosing spondylitis; acute and subacute bursitis; acute nonspecific tenosynovitis; acute gouty arthritis; post-traumatic osteoarthritis; synovitis of osteoarthritis; and epicondylitis. For example, the treatment may comprise an adjunctive therapy for short-term administration, wherein the patient in need thereof is having an acute episode or an exacerbation. In another example, selected methods, for example methods of treating rheumatoid arthritis, may require low-dose maintenance therapy.

Provided herein are compounds for use in treating collagen diseases. A pharmaceutically effective amount of a disclosed heterocyclic compound, e.g. a compound of Formula I, IA, IB, II, or IIA, is administered to a patient in need thereof. Contemplated compounds for use include, for example, treating systemic lupus erythematosus; systemic-dermatomyositis (e.g., polymyositis); and acute rheumatic carditis. For example, the treatment may be administered as maintenance therapy and/or when the patient in need thereof is having an acute episode or an exacerbation.

Also provided herein are compounds for use in treating dermatologic diseases. A pharmaceutically effective amount of a disclosed heterocyclic compound, e.g. a compound of Formula I, IA, IB, II, or IIA, is administered to a patient in need thereof. Contemplated treatments include, for example, treating pemphigus; bullous dermatitis herpetiformis; erythema multiforme, e.g., Stevens-Johnson syndrome; exfoliative dermatitis; mycosis fungoides; psoriasis; and seborrheic dermatitis.

Compounds for use in treating allergic conditions are provided. A pharmaceutically effective amount of a disclosed heterocyclic compound, e.g. a compound of Formula I, IA, IB, II, or IIA, is administered to a patient in need thereof. Contemplated treatments include, for example, treating seasonal or perennial allergic rhinitis; bronchial asthma; contact dermatitis; atopic dermatitis; serum sickness; and drug hypersensitivity reactions. For example, these include control of severe or incapacitating allergic conditions that are hard to control with conventional treatment.

Provided herein are compounds for treating ophthalmic diseases. A pharmaceutically effective amount of a disclosed heterocyclic compound, e.g. a compound of Formula I, IA, IB, II, or IIA, is administered to a patient in need thereof. Contemplated treatments include, for example, treating acute and chronic allergic and inflammatory processes involving the eye and/or its adnexa such as: allergic cornea marginal ulcers; herpes zoster ophthalmicus; anterior segment inflammation; uveitis, choroiditis, scleritis, episcleritis; sympathetic ophthalmia; allergic conjunctivitis; keratitis; chorioretinitis; optic neuritis; iritis and iridocyclitis.

Compounds for use in treating respiratory diseases are provided. A pharmaceutically effective amount of a disclosed heterocyclic compound, e.g. a compound of Formula I, IA, IB, II, or IIA, is administered to a patient in need thereof. Contemplated treatments include, for example, treating symptomatic sarcoidosis; Loeffler's syndrome; berylliosis; fulminating or disseminated pulmonary tuberculosis, for example, wherein the treatment can comprise of concurrent administration of a disclosed compound and an appropriate antituberculous chemotherapy; and aspiration pneumonitis.

Also provided herein are compounds for use in treating hematologic diseases. A pharmaceutically effective amount of a disclosed heterocyclic compound, e.g. a compound of Formula I, IA, IB, II, or IIA, is administered to a patient in need thereof. For example, contemplated treatments include treating idiopathic thrombocytopenic purpura; secondary thrombocytopenia; acquired (autoimmune) hemolytic anemia; erythroblastopenia (RBC anemia); and congenital (erythroid) hypoplastic anemia.

Provided herein are compounds for use in treating neoplastic diseases. A pharmaceutically effective amount of a disclosed heterocyclic compound, e.g. a compound of Formula I, IA, IB, II, or IIA, is administered to a patient in need thereof. Contemplated treatments include, for example, methods of treating leukemia and lymphoma, including a method of treating acute leukemia in children.

Also provided herein are compounds for use in treating edematous states. A pharmaceutically effective amount of a disclosed heterocyclic compound, e.g. a compound of Formula I, IA, IB, II, or IIA, is administered to a patient in need thereof. Contemplated treatments include, for example, inducing diuresis or remission of proteinuria in the nephrotic syndrome, without uremia, of the idiopathic type or that due to lupus erythematosus.

Provided herein are compounds for use in treating nervous system diseases and/or disorders. A pharmaceutically effective amount of a disclosed heterocyclic compound, e.g. a compound of Formula I, IA, IB, II, or IIA, is administered to a patient in need thereof. Contemplated methods include, for example, methods of treating multiple sclerosis.

Also provided herein are compounds for use in treating tuberculous meningitis with subarachnoid block or impending block. A pharmaceutically effective amount of a disclosed heterocyclic compound, e.g. a compound of Formula I, IA, IB, II, or IIA, used concurrently with appropriate antituberculous chemotherapy, is administered to a patient in need thereof. Also disclosed herein are compounds for use in treating trichinosis with neurologic or myocardial involvement. A pharmaceutically effective amount of a disclosed heterocyclic compound, e.g. a compound of Formula I, IA, IB, II, or IIA, is administered to a patient in need thereof.

### Pharmaceutical Compositions and Administration

The disclosure provides pharmaceutical compositions, which comprise a therapeutically-effective amount of one or more of the compounds described above, formulated together with one or more pharmaceutically acceptable carriers (additives) and/or diluents. The pharmaceutical compositions may be specially formulated for administration in solid or liquid form, including those adapted for the following: (1) oral administration, for example, drenches (aqueous or non-aqueous solutions or suspensions), tablets, e.g., those targeted for buccal, sublingual, and systemic absorption, boluses, powders, granules, pastes for application to the tongue; (2) parenteral administration, for example, by subcutaneous, intramuscular, intravenous or epidural injection as, for example, a sterile solution or suspension, or sustained-release formulation; (3) topical application, for example, as a cream, ointment, or a controlled-release patch or spray applied to the skin; (4) intravaginally or intrarectally, for example, as a pessary, cream or foam; (5) sublingually; (6) ocularly; (7) transdermally; or (8) nasally; or other formulations to be used as aerosols and/or nebulizers.

Another aspect provides pharmaceutical compositions which comprise a therapeutically effective amount of one or more of the disclosed compounds, formulated together with one or more other therapeutic agents, for example as part of a combination treatment.

In one embodiment, the pharmaceutical compositions of the present disclosure may be administered orally. However, the pharmaceutical compositions may be administered parenterally, by inhalation spray, topically, rectally, nasally, buccally, vaginally or via an implanted reservoir. Contemplated non-parenteral administration includes oral, buccal, transdermal (e.g., by a dermal patch), topical, inhalation, or sublingual administration, or, e.g., ocular, pulmonary, nasal, rectal or vaginal administration. For example, a contemplated pharmaceutical compositions may be administered orally, intraperitoneally or intravenously.

Sterile injectable forms of the pharmaceutical compositions may be aqueous or oleaginous suspensions. These suspensions may be formulated according to techniques known in the art, using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium.

For this purpose, any bland fixed oil may be employed including synthetic mono- or diglycerides. Fatty acids, such as oleic acid and its glyceride derivatives are useful in the preparation of injectables, as are natural pharmaceutically-acceptable oils such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers, which are commonly used in the manufacture of pharmaceutically acceptable solid, liquid, or other dosage forms may also be used for the purposes of formulation.

The pharmaceutical compositions of this disclosure may be orally administered in any orally acceptable dosage form including, but not limited to, solid forms such as capsules, tablets, pills, powders, and granules, which may be prepared using any suitable process known to the art. For example, a disclosed compound may be mixed with enteric materials and compressed into tablets. In the case of tablets for oral use, carriers commonly used include microcrystalline cellulose, lactose and corn starch. Lubricating agents, such as magnesium stearate, are also typically added.

Alternatively, formulations of the disclosure may be incorporated into chewable tablets, crushable tablets, tablets that dissolve rapidly within the mouth, or mouth wash.

Liquid dosage forms for oral or other administration include, but are not limited to, pharmaceutically acceptable emulsions, microemulsions, solutions, suspensions, syrups and elixirs. In addition to the active agent(s), the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the ocular, oral, or other systemically-delivered compositions can also include adjuvants such as wetting agents, and emulsifying and suspending agents. If desired, certain sweetening, flavoring or coloring agents may also be added.

The pharmaceutical compositions of this disclosure may also be administered by nasal aerosol or inhalation. Such pharmaceutical compositions are prepared according to techniques well known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

For example, for pulmonary (e.g., intrabronchial) administration, disclosed compounds can be formulated with conventional excipients to prepare an inhalable composition in the form of a fine powder or atomizable liquid. For ocular administration, disclosed compounds can be formulated with conventional excipients, for example, in the form of eye drops or an ocular implant. Among excipients useful in eye drops are viscosifying or gelling agents, to minimize loss by lacrimation through improved retention in the eye.

Should topical or transdermal administration be desired, it can be accomplished using any method commonly known to those skilled in the art. Dosage forms for topical or transdermal administration of an inventive pharmaceutical composition includes but is not limited to ointments, pastes, creams, lotions, gels, powders, solutions, sprays, inhalants, or patches. The active agent is admixed under sterile conditions with a pharmaceutically acceptable carrier and any needed preservatives or buffers as may be required. For example, cutaneous routes of administration are achieved with aqueous drops, a mist, an emulsion, or a cream.

Transdermal patches may have the added advantage of providing controlled delivery of the active ingredients to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

Compositions for rectal or vaginal administration may be suppositories, which can be prepared by mixing a disclosed compound with suitable non-irritating excipients or carriers such as cocoa butter, polyethylene glycol or a suppository wax which are solid at ambient temperature but liquid at body temperature and therefore melt in the rectum or vaginal cavity and release the active agent(s). Alternatively, contemplated formulations can be administered by release from a lumen of an endoscope after the endoscope has been inserted into a rectum of a subject.

The amount of the disclosed compound that may be combined with the carrier materials to produce a pharmaceutical composition in a single dosage form will vary depending upon the host treated and the particular mode of administration. It should be understood that a specific dosage and treatment regimen for any particular patient will depend upon a variety of factors, including the activity of the specific compound employed, the age, body weight, general health, sex, diet, time of administration, rate of excretion, drug combination, and the judgment of the treating physician as well as the severity of the particular disease being treated. Despite their variety, accounting for these factors in order to select an appropriate dosage or treatment regimen would require no more than routine experimentation.

Additional agents, i.e., agents other than the disclosed compounds, contemplated herein may be administered separately from the therapeutic agents of the disclosure, as part of a multiple dosage regimen. Alternatively, those agents may be part of a single dosage form, mixed together with the therapeutic agents of the disclosure in a single pharmaceutical composition. If administered as part of a multiple dosage regime, the two active agents may be administered simultaneously, sequentially or within a period of time from one another. The amount of both the therapeutic agent, i.e., a disclosed compound, of the disclosure and the additional therapeutic agent that may be combined with the carrier materials to produce a single dosage form will vary depending upon the host treated and the particular mode of administration as well as on the nature of the therapeutic agent of the disclosure and the additional therapeutic agent.

Contemplated methods may include administration of a composition comprising a disclosed compound, for example, hourly, twice hourly, every three to four hours, daily, twice daily, 1, 2, 3 or 4 times a week, every three to four days, every week, or once every two weeks depending on half-life and clearance rate of the particular composition or inhibitor.

Treatment can be continued for as long or as short a period as desired. The compositions may be administered on a regimen of, for example, one to four or more times per day. A suitable treatment period can be, for example, at least about one week, at least about two weeks, at least about one month, at least about six months, at least about 1 year, or indefinitely. For example, the treatment period can be at least about one month, or at least about six months, or at least about one year, or indefinitely when treating a metabolic disorder.

### EXAMPLES

The disclosure is further illustrated by the following examples.

### Example 1 : Compound S3.1

Compound S3.1 is synthesized according to the following scheme:

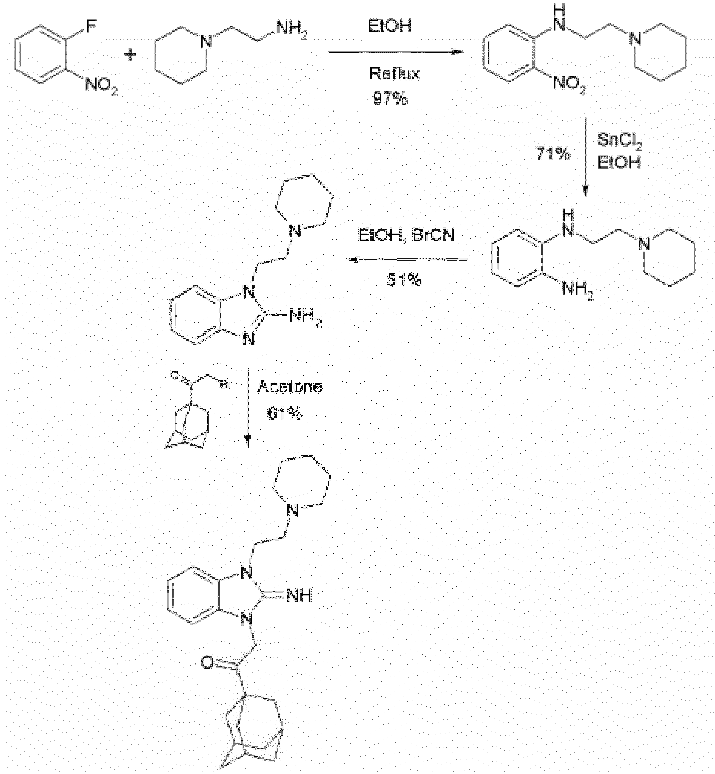

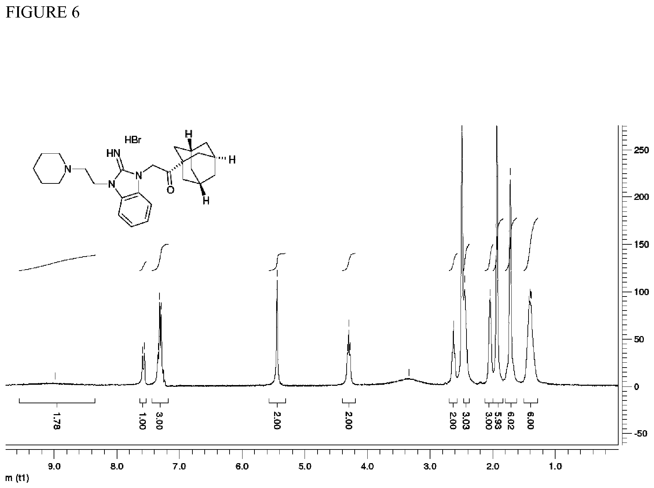

The NMR spectra is shown in Figure 6.

### Example 2 1-(2-cyclohexyl-2-oxoethyl)-3-(2-(piperidin-1-yl)ethyl)-1H-benzo[d]imidazol-2(3H)-one

The named compound, below, is prepared using the following scheme:

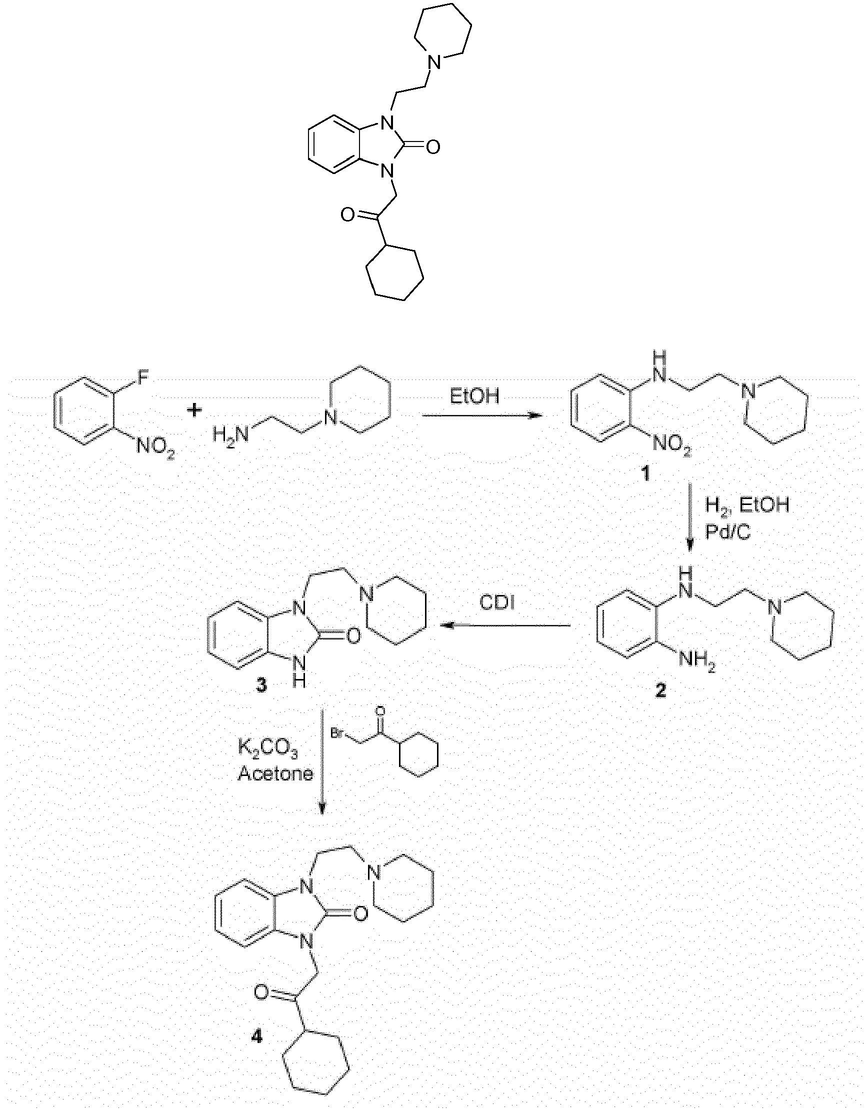

The first step, S_{N}Ar reaction was realized in 74% yield. The nitro reduction to give compound 2 is almost quantitative. The cyclisation of compound 3 was realised in good yield. The final alkylation yielded product 4 in 23% yield.

### Example 3:

The compound is prepared using the following scheme:

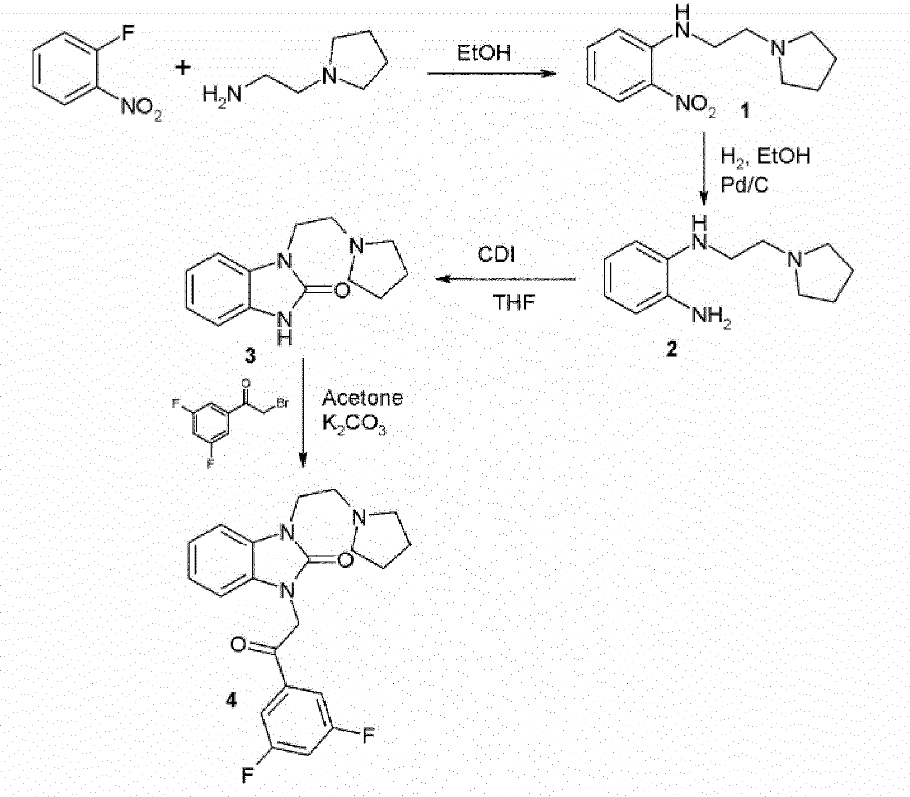

The first S_{N}Ar reaction was realized in good yield to give compound 1. The nitro reduction was carried out under hydrogenation in nearly quantitative yield. The bisaniline 2 was then engaged in a cyclisation to form the benzimidazole ring 3. This reaction is also highly reproducible in good yields. The alkylation was relaunched several times on a larger scale and after purification, a sufficient quantity of product was recovered clean.

### Example 4

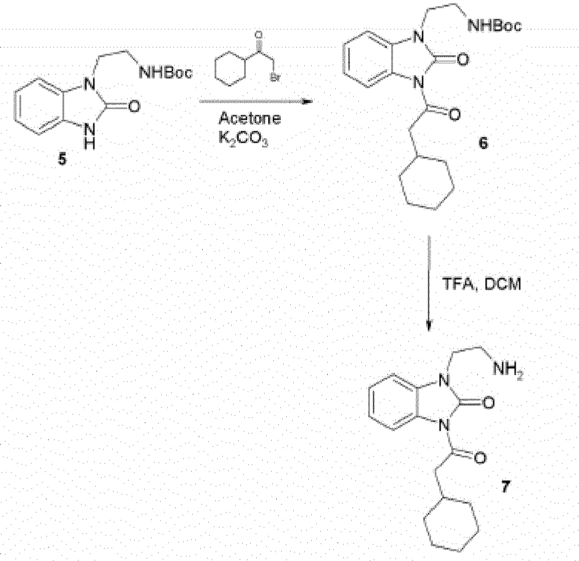

Starting from the benzimidazolone 5 obtained during the synthesis of compound 22187, the alkylation was realised in 42% yield. The product was used in a Boc deprotection step to yield compound 7.

### Example 5

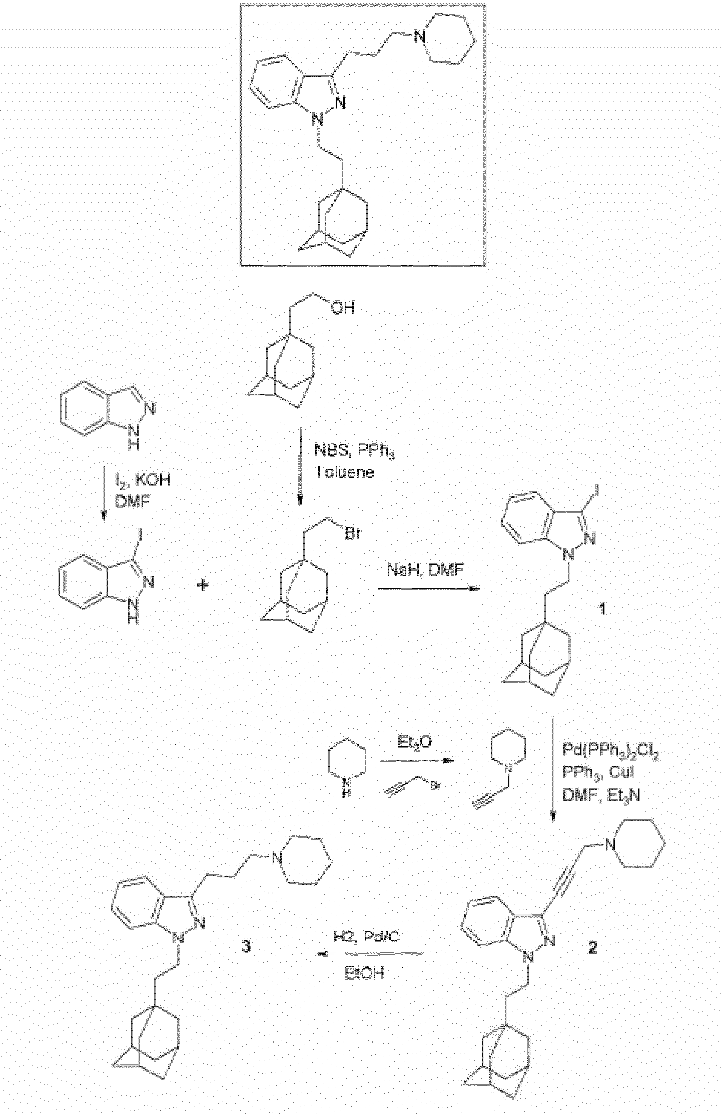

The synthesis has started with the electrophilic iodination of the indazole. The product was obtained in 87% yield after purification. In parallel, the adamantane ethyl bromide was prepared from the alcohol derivative in good yield. Deprotonation/alkylation of the indazole allowed the recovery of the desired product 1 in 68% yield. The propargyl piperidine was also prepared in a 62% yield. The Sonogashira was realized under standard conditions to give compound 2 in 82% yield. The reduction under hydrogenation conditions yielded the final product 3 in 55% yield after purification.

### Example 6

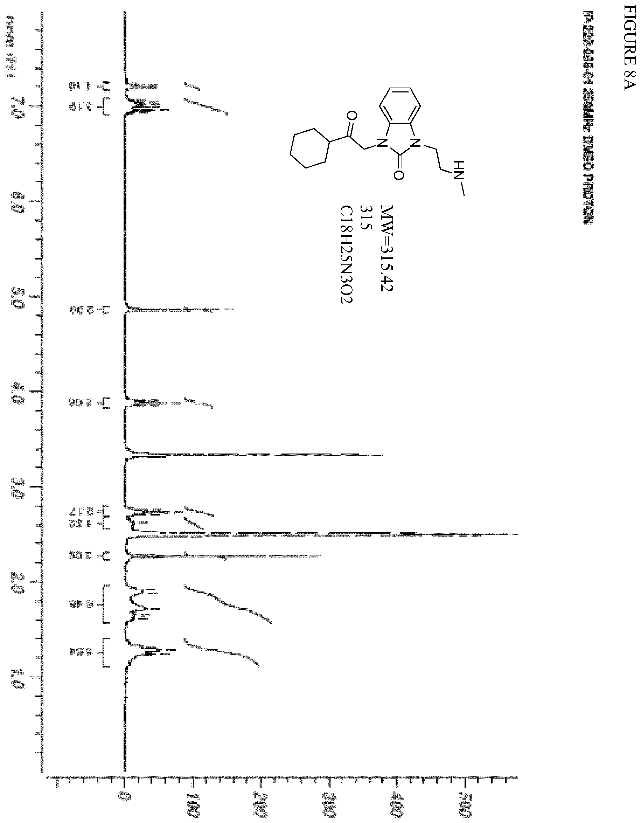

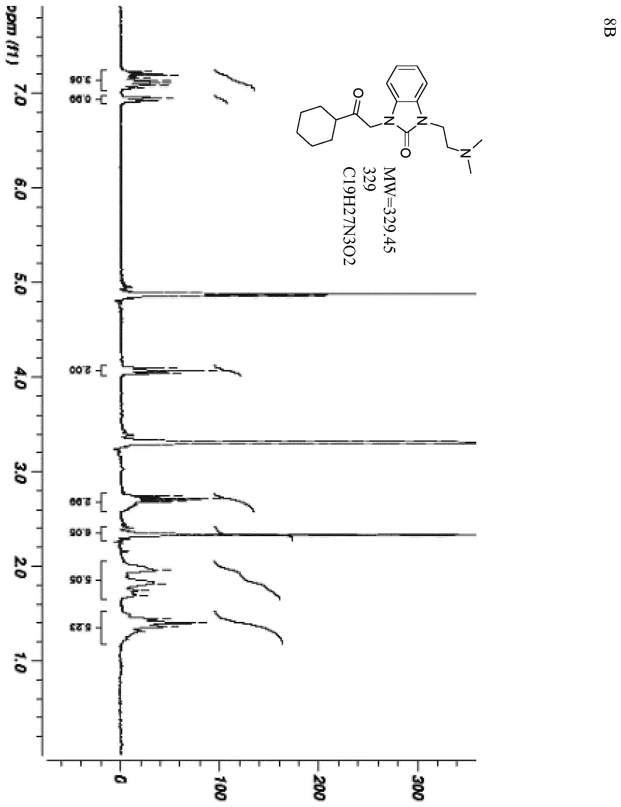

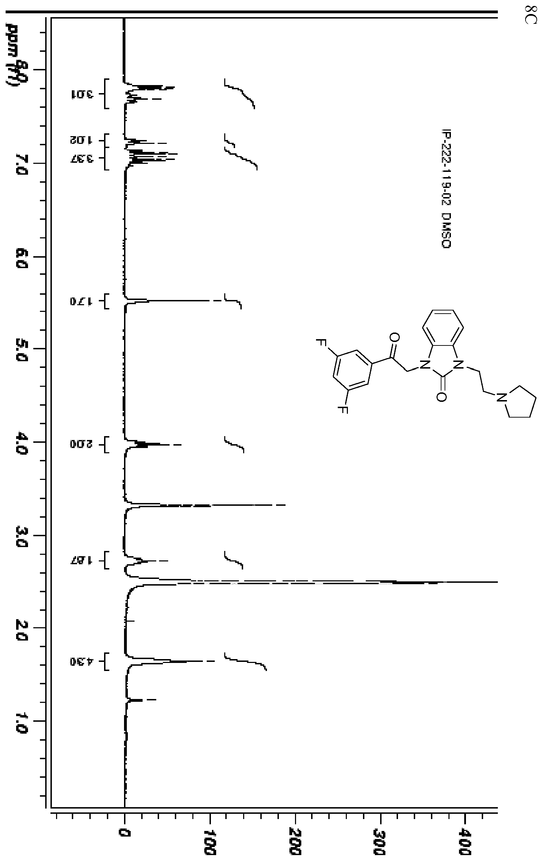

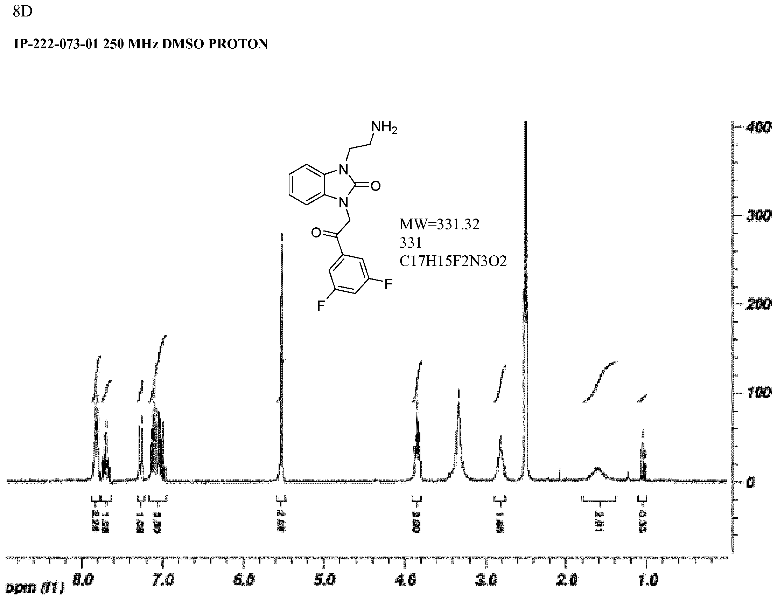

Compounds listed in Table 1 below were prepared using procedures analogous to those described herein. NMR spectra are depicted in Figure 8.

| **Example No.** | **Compound Structure** | **MS Calc. M⁺** | **MS Found M⁺+1** | **Retention Time** |
|---|---|---|---|---|
| **1** | 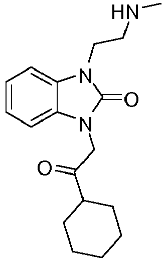 | 315.42 | 316.2 | 4.82 min |
| **2** | 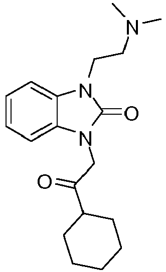 | 329.45 | 330.2 | 5.11 min |
| **3** | 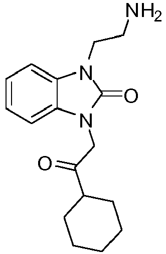 | 301.39 | 302.1 | 3.53 min |
| **4** | 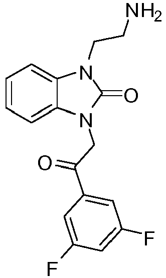 | 331.32 | 332.1 | 4.67 min |
| **5** | 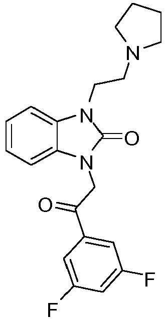 | 385.42 | 386.2 | 3.29 min |
| **6** | 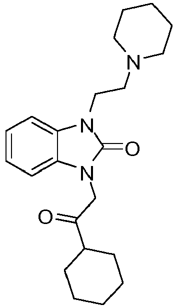 | 369.51 | 370.2 | 5.36 min |
| 7 | 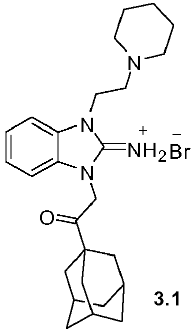 | 420.60 | 421.2 | 3.21 min |
| **8** | 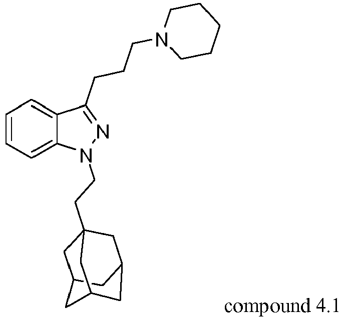 | 405.63 | 406.2 | 7.26 min |
| | 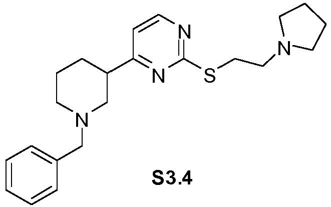 | | | |
| | 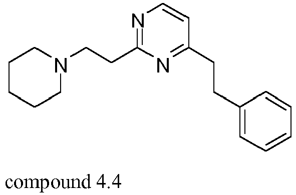 | | | |

### Example 7: Inhibition of T Lymphocyte Proliferation

Select compounds were screened for their ability to inhibit T lymphocyte proliferation.

Human peripheral blood lymphocytes (PBLs) were isolated from whole blood of healthy volunteers by Ficoll-Isopaque density gradient centrifugation. After washing, monocytes were separated from lymphocytes by percoll density gradient centrifugation. The lymphocytes were cultured in IMDM (Gibco, Verviers, Belgium) supplemented with 10% heat inactivated fetal calf serum.

Cells were seeded in 96 well plates and stimulated with PHA (10 µg/ml) and corresponding concentration of pharmaceuticals. After 24 hours tritiated thymidine was added to each well and left overnight. Next morning proliferation was measured by microbeta counter. Graphs were plotted with the untreated condition set to a hundred procent.

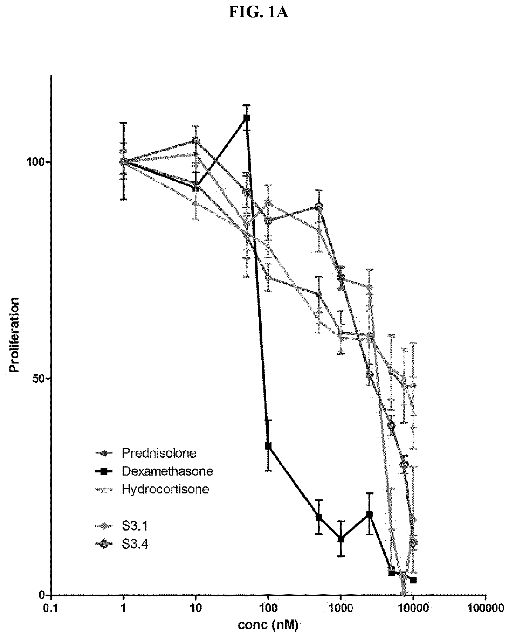

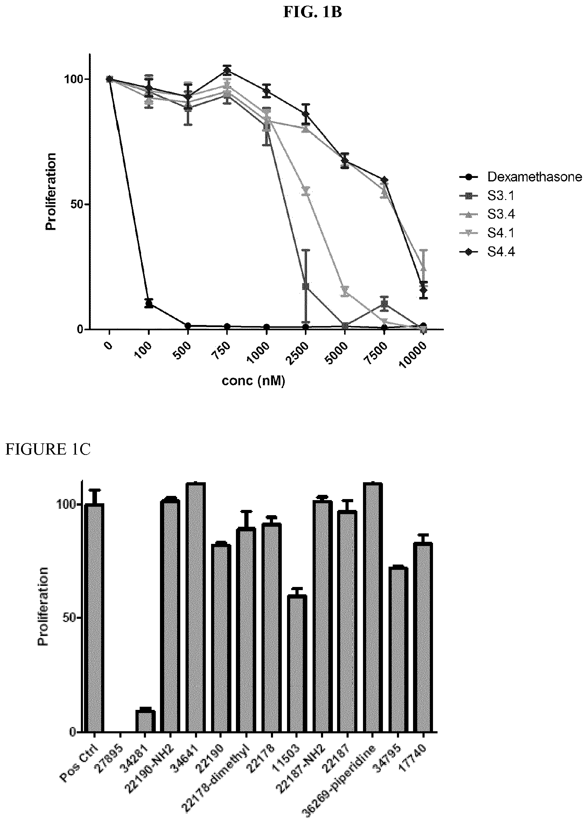

As shown in Fig. 1A and 1B, the antiproliferative effect of the compounds S3.1, S3.4, S4.1, and S4.4 is compared to glucocorticoids, such as prednisolone, dexamethasone, and hydrocortisone. To test the potential of the compounds, inhibition of proliferation was plotted against known corticosteroids (Fig 1A). Dexamethasone is the most powerful corticosteroid used in this experiment and also one of the most potent anti-inflammatory drugs known. Prednisolone is a more commonly used corticosteroid and hydrocortisone is the equivalent of the steroids produced in the adrenal cortex.

Figure 1B compares inhibition of lymphocyte proliferation of the compounds (S4.1 and S4.4) with compounds (S3.1 and S3.4) at different concentrations.

*In vitro* stimulation of lymphocytes using phytohematoglutinin (PHA) was compared to superantigen Enterotoxin B (SEB) stimulated T lymphocytes. Compounds were evaluated for their ability to fully suppress PHA stimulated lymphocyte proliferation and SEB stimulated proliferation partially.

Lymphocytes were seeded in a 6 well plate (1,0·10⁷/well), pretreated for 10 minutes with S3.1, S3.4 or dexamethasone and stimulated for 10 minutes with PHA. The experiment was stopped by adding a large volume of ice cold PBS to the cells. The suspension was transferred to a tube, briefly spinned down in a centrifuge and the supernatant removed. The pellet was lysed by adding cold lysisbuffer (0,1% NP40, 50mM TrisHCl, 1mM EDTA, 1mM EGTA, 150mM NaCl, 200µM Na₃VO₄, 10mM NaF and protease inhibitors) and incubating 20 minutes on ice.

To investigate the cell signaling events that are affected by treatment with S3.1 and S3.4, lysates were supplemented with sample buffer (188 mM Tris-Cl (pH 6.8), 3% SDS, 30% glycerol, 0.01% bromophenol blue, 15% β-mercaptoethanol), heated to 95°C for 5 minutes, cooled on ice and sonicated. Samples were then run on 10% SDS-PAGE gel, blotted to PVDF membrane and incubated with antibodies for phospho-Zap70 (Cell Signaling Technology (Beverly, MA)) and phospho-LAT (Cell Signaling Technology (Beverly, MA)). To show equal loading of the samples the blot was incubated with an antibody directed against beta-actin (Santa Cruz Biotechnology (Heidelberg, Germany)).

The kinase Lck acts as a gate keeper to T cell receptor (TCR) related signaling as it is one of the first and most up stream kinases to be activated upon stimulation of the TCR. As downstream signaling of the TCR is inhibited by S3.1 and S3.4 treatment interactions with Lck were investigated by immunoprecipitating Lck and blotting for Hsp90. For immunprecipitation experiments 1,0·10⁷ cells were used per condition. Cells were pelleted and lysed as described previously. The supernatant was precleared by adding 10 µl Protein A/G UltraLink resin (Pierce Biotechnology, Rockford, USA) for 30 minutes at 4°C. Beads were spinned down and discarded. Samples were incubated with 2 µg primary antibody and 25 µl beads for 2 hours at 4°C. Beads were washed with PBS, sample buffer was added and heated to 95°C for 5 minutes.

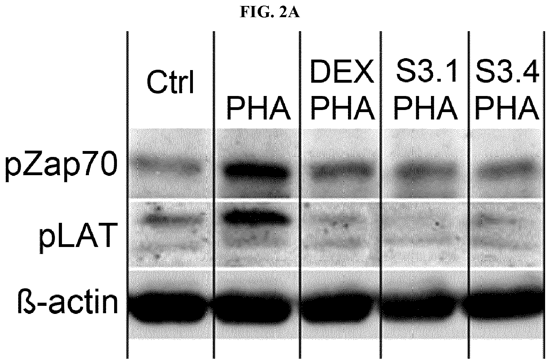

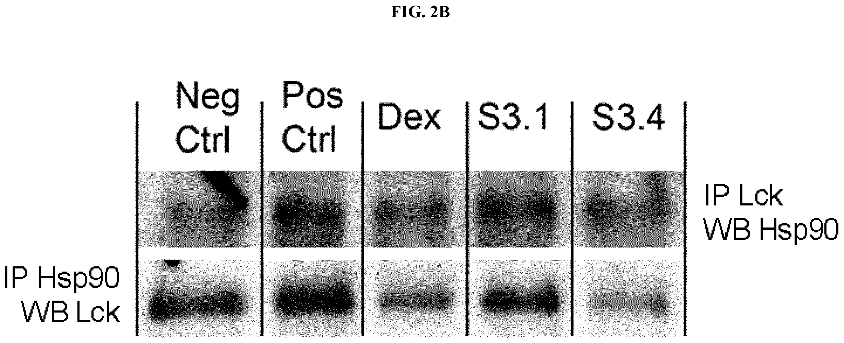

The antiproliferative effect of compounds S3.1 and S3.4 is further reflected in the loss of T-Cell receptor (TCR) related signaling as was investigated by westernblot (see Fig. 2A and 2B). Both the kinase Zap70 and the scaffolding protein LAT showed lower phosphorylation levels compared to a stimulated untreated control (Fig. 2A). Similar to the glucocorticoid (GC), dexamethasone treatment the interaction between lymphocyte-specific protein tyrosine kinase (LCK) and its chaperone Hsp90 was lost (Fig. 2B). This interaction protects LCK from degradation. Taken together this confirms an effect on TCR-proximal signaling, affecting LCK and downstream signaling events which ultimately result in the loss of proliferation and consequently a reduction of cytokine levels.

### Example 8: Toxicity Evaluation (MTS Viability Assay on HELA cervical cancer cells)

Compound S3.1 and S3.4 were tested against multiple cancer cell lines (HCT116, Hela, 293T) to assess toxicity using a MTS viability assay. Cell lines were treated for three days at 10 µM. Cells were seeded at multiple concentrations in a 96 wells plate in 100 µl medium. Next day S3.1 or S3.4 was added to each well at a concentration of 10 µM. To test cell viability, 20 µl of MTS solution (Promega, Madison, USA) was added to each well and incubated for four hours. Formation of the blue formazan was determined by measuring absorbance at 490nm using a 96-well plate reader. Formazan is produced as MTS is reduced by the cells. Turnover of MTS correlates with the viability of the cells.

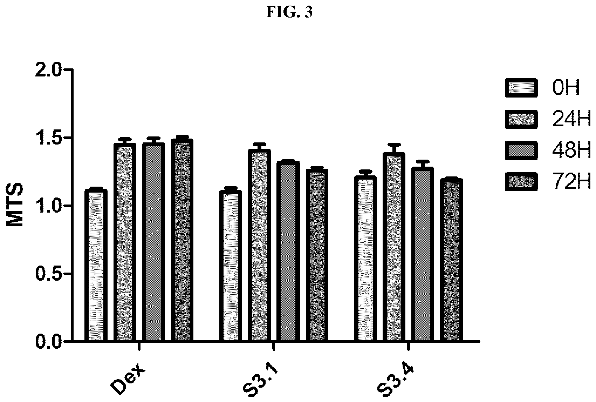

As seen in Fig. 3, the compounds did not result in any negative effects on viability or proliferation.

### Example 9: Comparison to Glucocorticoids and Evaluation of Side Effects

Because the compounds were selected for an effect similar to that of GC treatment and GC treatment is known to be accompanied by severe unwanted side effects, the compounds were screened for their ability to induce GC regulated genes. The tests were performed using a cell line which contains a GRE-luciferase reporter construct. GRE refers to glucocorticoid responsive element, a specific DNA sequence to which the ligand bound glucocorticoid receptor binds and luciferase is a gene coding for a protein which can catalyze the oxidation of luciferin, a reaction which is accompanied by bioluminescence.

293 GRE cancer cells were purchased from Panomics (Vignate-Milano, Italy). Cells were seeded in a 24 well plate at 1·10⁵ cells/well. After 48 hours cells were treated overnight with compounds or dexamethasone. Wells were aspirated and the cells lysed by adding 100 µl lysisbuffer (Promega, Madison, USA) to each well. 10 µl of the lysate was added to 100 µl of luciferase substrate. Produced light was measured by luminometer. To show that there is no transactivation of GR regulated genes (as would be the case with generic GR ligands) two well known side effects of corticosteroid treatment were investigated.

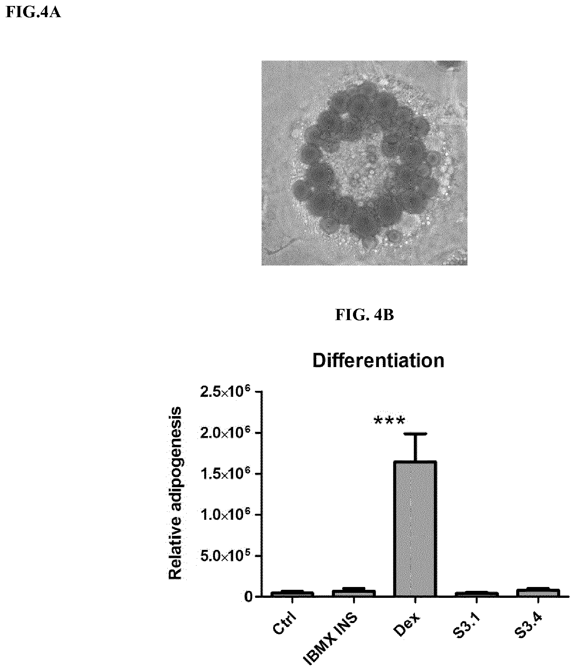

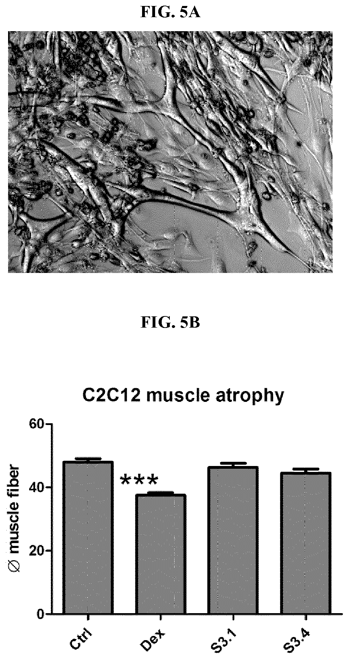

Fig4a/4b Adipogenesis: Figures 4A/4B shows results after 3T3-L1 mouse fibroblasts were cultured in DMEM +10%FCS. After reaching full confluency the media was supplemented with insulin (1,6µM), IBMX (0,5mM) and dexamethasone or S3.1 or S3.4 (0,25µM). After two days the media was replaced by growth medium containing insulin. After 6 days this medium was replaced by growth medium. A week later formation of lipid droplets was visualized by Oil Red O staining and quantified. Briefly; Cells were fixed for 15 minutes with 4% paraformaldehyde, washed with PBS, incubated in 4,4mM Oil Red O/isopropanol solution for 10 minutes, washed 2x 10 minutes with H₂O and mounted in glycerol. Fig5a/5b shows results after Muscle atrophy C2C12 mouse myoblasts were cultured in DMEM +5%FCS. When cells reached 90% confluency replace medium with DMEM supplemented with 5%HS and 1µM insulin. After two days treat the cells with 4µg/ml AraC. After one week cells were treated for 24 hours with 10 µM dexamethasone or S3.1 or S3.4. The experiment was stopped by fixing the cells overnight in cold 2% glutaraldehyde solution. This fixation method causes auto fluorescence allowing easy visualization of the cells. Thickness of the formed muscle fibers was measured and quantified using Adobe photoshop CS4.

In the *in vitro* mouse myoblast cell line C2C12 assay, none of the compounds induce differentiation of fibroblasts into adipocytes (Fig. 4A and 4B) or cause loss of muscle fiber thickness (Fig. 5A and 5B), each of which are reported side effects of GC treatment.

The invention may be embodied in other specific forms without departing from the essential characteristics thereof. The foregoing embodiments are therefore to be considered in all respects illustrative rather than limiting on the invention described herein. Scope of the invention is thus indicated by the appended claims rather than by the foregoing description, and all changes that come within the meaning and range of the claims are intended to be embraced therein.

## Claims

1. A heterocyclic compound represented by: 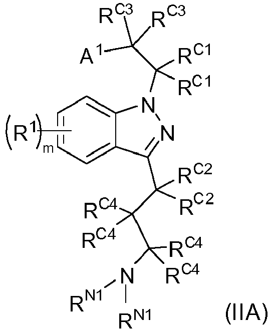 wherein
A¹ is selected from the group consisting of C₃₋₆cycloalkyl, heterocyclyl, phenyl, naphthyl, heteroaryl, C₁₋₆alkyl, hydroxy, -NR'R', -O-C₁₋₆alkyl, -S-C₁₋₆alkyl, - U-C₃₋₆cycloalkyl, -U-heterocyclyl, -U-phenyl, -U-naphthyl, -U-heteroaryl, phenylalkyl, naphthylalkyl, heterocycloalkyl, and heteroarylalkyl; wherein C₃₋₆cycloalkyl, heterocyclyl, phenyl, naphthyl, heteroaryl, and C₁₋₆alkyl are optionally substituted with one, two, three or more substituents each selected from R^{A1};
U is selected from the group consisting of -S-, -NR'-, -O-, or C₁₋₆alkyl;
R^{C1}, R^{C2}, R^{C3} and R^{C4} are independently selected, for each occurrence, from the group consisting of hydrogen, halogen, hydroxyl, amino, amido, sulfonyl, sulfonamide, thiol, C₁₋₆alkyl, haloC₁₋₆alkyl, carboxyl, cyano, nitro, alkoxy, C₃₋₆cycloalkyl, heterocyclyl, phenyl, naphthyl, and heteroaryl;
or two R^{C3} substituents or two R^{C4} substituents may be taken together to form an oxo, imino or sulfanylidene;
or two R^{C1} substituents, two R^{C2} substituents, two R^{C3} substituents or two R^{C4} substituents may be taken together with the atom or atoms to which they are attached to form a C₃₋₆cycloalkyl, phenyl, naphthyl, or a saturated, partially saturated or unsaturated, 4-6 membered monocyclic or 10-12 membered bicyclic heterocycle having one, two or three heteroatoms independently selected from the group consisting of N, O, and S;
or one R^{C1} substituent and one R^{C3} substituent or one R^{C2} substituent and one R^{C4} substituent may be taken together with the atoms to which they are attached to form a C₃₋₆cycloalkyl, phenyl, naphthyl, or a saturated, partially saturated or unsaturated, 4-6 membered monocyclic or 10-12 membered bicyclic heterocycle having one, two or three heteroatoms independently selected from the group consisting of N, O, and S;
R¹ is independently selected, for each occurrence, from the group consisting of halogen, hydroxyl, amino, amido, sulfonyl, sulfonamide, thiol, C₁₋₆alkyl, haloC₁₋₆alkyl, carboxyl, -C(O)O-C₁₋₆alkyl, -OC(O)O-C₁₋₆alkyl, -OC(O)NR'R', -N(R')C(O)NR'R', -N(R')C(O)O-C₁₋₆alkyl, cyano, nitro, alkoxy, C₃₋₆cycloalkyl, heterocyclyl, heteroaryl and phenyl;
R^{A1} is independently selected, for each occurrence, from the group consisting of halogen, hydroxyl, amino, amido, sulfonyl, sulfonamide, thiol, C₁₋₆alkyl, haloC₁₋₆alkyl, carboxyl, cyano, nitro, alkoxy, -C(O)O-C₁₋₆alkyl, -OC(O)O-C₁₋₆alkyl, -OC(O)NR'R', -N(R')C(O)NR'R', -N(R')C(O)O-C₁₋₆alkyl, oxo, C₃₋₆cycloalkyl, heterocyclyl, heteroaryl and phenyl;
R^{N1} and R' are independently selected, for each occurrence, from the group consisting of hydrogen and C₁₋₆alkyl; wherein C₁₋₆alkyl is optionally substituted with one, two, three or more substituents independently selected, for each occurrence, from R^{A1};
or two R^{N1} substituents or two R' substituents may be taken together with the nitrogen to which they are attached to form a saturated, partially saturated or unsaturated, 4-7 membered monocyclic or 10-15 membered bicyclic heterocycle having one, two or three heteroatoms independently selected from the group consisting of N, O, and S;
or one R^{N1} substituent and one R^{C4} substituent may be taken together with the atoms to which they are attached to form a saturated, partially saturated or unsaturated, 4-7 membered monocyclic or 10-15 membered bicyclic heterocycle having one, two or three heteroatoms independently selected from the group consisting of N, O, and S;
m is selected from the group consisting of 0, 1, 2 and 3; or
a pharmaceutically acceptable salt or a stereoisomer thereof.

2. The heterocyclic compound of claim 1, wherein A¹ is C₃₋₆cycloalkyl.

3. The heterocyclic compound of claim 2, wherein A¹ is selected from the group consisting of cyclohexyl, cyclohexenyl, cyclopentyl, cyclopentenyl, cyclobutyl, cyclopropyl, and any bridged, spiro or fused variant thereof.

4. The hetereocyclic compound of any one of claims 1 to 3, wherein 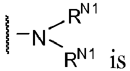 selected from the group consisting of: 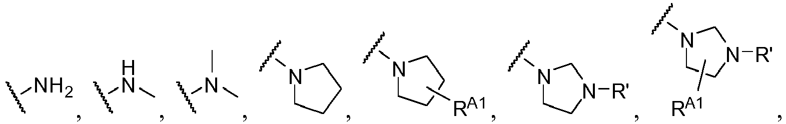 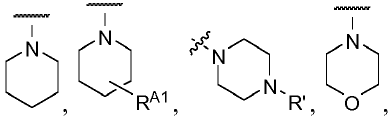 and

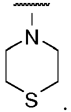

5. A heterocyclic compound selected from the group consisting of: 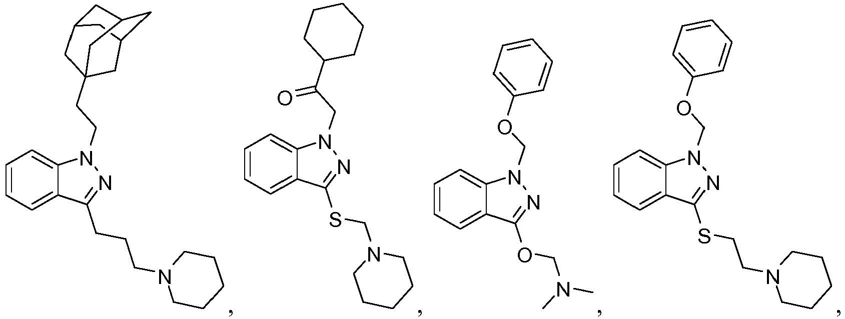 and 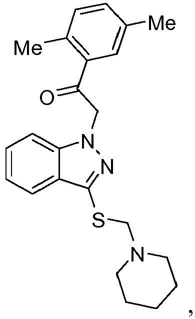 or a pharmaceutically acceptable salt thereof.

6. The heterocyclic compound of claim 5, wherein the heterocyclic compound is 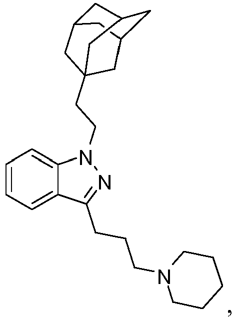 or a pharmaceutically acceptable salt thereof.

7. A compound represented by: 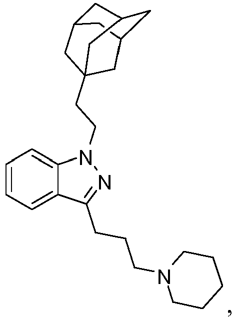 or a pharmaceutically acceptable salt thereof, for use in the treatment of an immune inflammatory disease or disorder.

8. A compound for use according to claim 7, wherein the immune inflammatory disorder is selected from the group consisting of Crohn's disease, ulcerative colitis, a rheumatic disorder, psoriasis, and an allergy.

9. A compound for use according to claim 8, wherein the allergy is contact dermatitis or atopic dermatitis.

10. A pharmaceutical composition comprising a compound according to any one of claims 1 to 6 and a pharmaceutically acceptable adjuvant or excipient.

## Patentansprüche

1. Heterozyklische Verbindung, die dargestellt ist durch: 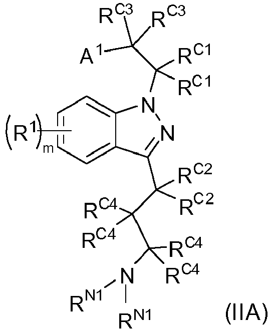 wobei
A¹ aus der Gruppe ausgewählt ist, die aus Folgenden besteht: C₃₋₆ Cycloalkyl, Heterocyclyl, Phenyl, Naphthyl, Heteroaryl, C₁₋₆ Alkyl, Hydroxy, -NR'R', -O-C₁₋₆ Alkyl, -S-C₁₋₆ Alkyl, -U-C₃₋₆ Cycloalkyl, -U-Heterocyclyl, -U-Phenyl, -U-Naphthyl, -U-Heteroaryl, Phenylalkyl, Naphthylalkyl, Heterocycloalkyl und Heteroarylalkyl; wobei C₃₋₆ Cycloalkyl, Heterocyclyl, Phenyl, Naphthyl, Heteroaryl und C₁₋₆ Alkyl optional mit ein, zwei, drei oder mehr Substituenten substituiert sind, und zwar jeweils ausgewählt aus R^{A1};
U aus der Gruppe ausgewählt ist, die aus Folgenden besteht: -S-, - NR'-, -O- oder C₁₋₆ Alkyl;
R^{C1}, R^{C2}, R^{C3} und R^{C4} unabhängig ausgewählt sind, für jedes Vorkommen, aus der Gruppe, die aus Folgenden besteht: Wasserstoff, Halogen, Hydroxyl, Amino, Amido, Sulfonyl, Sulfonamid, Thiol, C₁₋₆ Alkyl, Halo C₁₋₆ Alkyl, Carboxyl, Cyano, Nitro, Alkoxy, C₃₋₆ Cycloalkyl, Heterocyclyl, Phenyl, Naphthyl und Heteroaryl;
oder zwei R^{C3} Substituenten oder zwei R^{C4} Substituenten zusammengenommen werden können, um eine Oxo-, Imino- oder Sulfanyliden-Gruppe zu bilden;
oder zwei R^{C1} Substituenten, zwei R^{C2} Substituenten, zwei R^{C3} Substituenten oder zwei R^{C4} Substituenten mit dem Atom oder den Atomen, an die sie gebunden sind, zusammengenommen werden können, um ein C₃₋₆ Cycloalkyl, Phenyl, Naphthyl oder einen gesättigten, teilweise gesättigten oder ungesättigten 4-6 gliedrigen monozyklischen oder 10-12 gliedrigen bizyklischen Heterocyclus mit ein, zwei oder drei Heteroatomen, die unabhängig aus der Gruppe ausgewählt sind, die aus N, O und S besteht, zu bilden;
oder ein R^{C1} Substituent und ein R^{C3} Substituent oder ein R^{C2} Substituent und ein R^{C4} Substituent mit den Atomen, an die sie gebunden sind, zusammengenommen werden können, um ein C₃₋₆ Cycloalkyl, Phenyl, Naphthyl oder einen gesättigten, teilweise gesättigten oder ungesättigten 4-6 gliedrigen monozyklischen oder 10-12 gliedrigen bizyklischen Heterocyclus mit ein, zwei oder drei Heteroatomen, die unabhängig aus der Gruppe ausgewählt sind, die aus N, O und S besteht, zu bilden;
R¹ unabhängig ausgewählt ist, für jedes Vorkommen, aus der Gruppe bestehend aus Halogen, Hydroxyl, Amino, Amido, Sulfonyl, Sulfonamid, Thiol, C₁₋₆ Alkyl, Halo C₁₋₆ Alkyl, Carboxyl, -C(O)O-C₁₋₆ Alkyl, -OC(O)O-C₁₋₆ Alkyl, -OC(O)NR'R', -N(R')C(O)NR'R', -N(R')C(O)O-C₁₋₆ Alkyl, Cyano, Nitro, Alkoxy, C₃₋₆ Cycloalkyl, Heterocyclyl, Heteroaryl und Phenyl;
R^{A1} unabhängig ausgewählt ist, für jedes Vorkommen, aus der Gruppe bestehend aus Halogen, Hydroxyl, Amino, Amido, Sulfonyl, Sulfonamid, Thiol, C₁₋₆ Alkyl, Halo C₁₋₆ Alkyl, Carboxyl, Cyano, Nitro, Alkoxy, -C(O)O-C₁₋₆ Alkyl, -OC(O)O-C₁₋₆ Alkyl, -OC(O)NR'R', -N(R')C(O)NR'R', -N(R')C(O)O-C₁₋₆ Alkyl, Oxo, C₃₋₆ Cycloalkyl, Heterocyclyl, Heteroaryl und Phenyl;
R^{N1} und R' unabhängig ausgewählt sind, für jedes Vorkommen, aus der Gruppe bestehend aus Wasserstoff and C₁₋₆ Alkyl; wobei C₁₋₆ Alkyl optional substitutiert ist mit ein, zwei drei oder mehr Substituenten, die unabhängig ausgewählt sind für jedes Vorkommen, aus R^{A1};
oder zwei R^{N1} Substituenten oder zwei R' Substituenten mit dem Stickstoff, an den sie gebunden sind, zusammengenommen werden können, um einen gesättigten, teilweise gesättigten oder ungesättigten 4-7 gliedrigen monozyklischen oder 10-15 gliedrigen bizyklischen Heterozyklus zu bilden, der ein, zwei oder drei Heteroatome hat, die unabhängig aus der Gruppe ausgewählt sind, die aus N, O und S besteht;
oder ein R^{N1} Substituent und ein R^{C4} Substituent mit den Atomen, an die sie gebunden sind, zusammengenommen werden können, um einen gesättigten, teilweise gesättigten oder ungesättigten 4-7 gliedrigen monozyklischen oder 10-15 gliedrigen bizyklischen Heterozyklus zu bilden, der ein, zwei oder drei Heteroatome hat, die unabhängig aus der Gruppe ausgewählt sind, die aus N, O und S besteht;
m aus der Gruppe ausgewählt ist, die aus 0, 1, 2 und 3 besteht; oder
ein pharmazeutisch verträgliches Salz oder ein Stereoisomer davon.

2. Heterozyklische Verbindung nach Anspruch 1, wobei A¹ C₃₋₆ Cycloalkyl, ist.

3. Heterozyklische Verbindung nach Anspruch 2, wobei A¹ aus der Gruppe ausgewählt ist, die aus Cyclohexyl, Cyclohexenyl, Cyclopentyl, Cyclopentenyl, Cyclobutyl, Cyclopropyl und irgendeiner verbrückten, Spiro- oder kondensierten Variante davon besteht.

4. Heterozyklische Verbindung nach einem der Ansprüche 1 bis 3, wobei 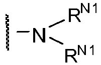 aus der Gruppe aus gewählt ist, die aus Folgenden besteht: 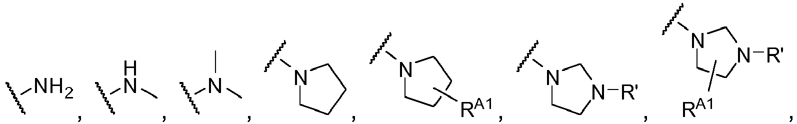 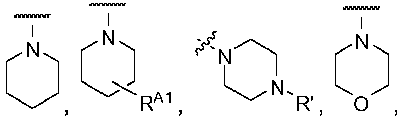 und

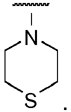

5. Heterozyklische Verbindung, die aus der Gruppe aus gewählt ist, die aus Folgenden besteht: 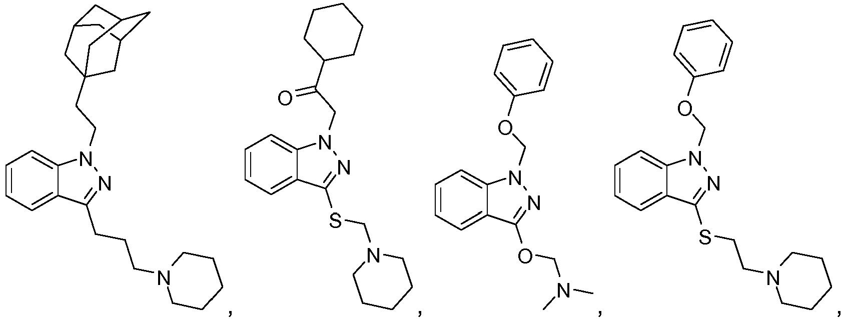 und 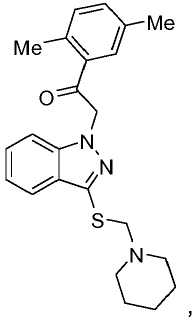 oder ein pharmazeutisch verträgliches Salz davon.

6. Heterozyklische Verbindung nach Anspruch 5, wobei die heterozyklische Verbindung Folgendes ist 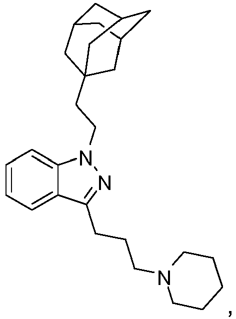 oder ein pharmazeutisch verträgliches Salz davon.

7. Verbindung, die dargestellt ist durch: 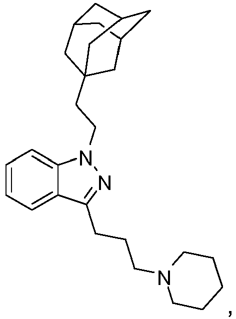 oder ein pharmazeutisch verträgliches Salz davon zur Verwendung bei der Behandlung einer entzündlichen Immunerkrankung oder Störung.

8. Verbindung zur Verwendung nach Anspruch 7, wobei die entzündliche Immunerkrankung aus der Gruppe ausgewählt ist, die aus Morbus Crohn, Colitis ulcerosa, einer rheumatischen Erkrankung, Psoriasis, und einer Allergie besteht.

9. Verbindung zur Verwendung nach Anspruch 8, wobei die Allergie eine Kontaktdermatitis oder eine atopische Dermatitis bzw. Neurodermitis ist.

10. Pharmazeutische Zusammensetzung, die eine Verbindung nach einem der Ansprüche 1 bis 6 aufweist und ein pharmazeutisch verträgliches Adjuvans oder einen Arzneimittelträger.

## Revendications

1. Composé hétérocyclique représenté par : 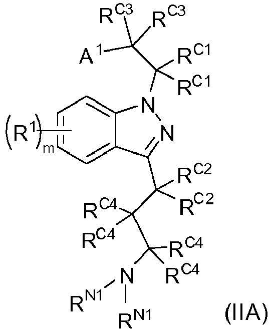 dans lequel
A¹ est sélectionné dans le groupe constitué de C₃₋₆cycloalkyle, hétérocyclyle, phényle, naphthyle, hétéroaryle, C₁₋₆alkyle, hydroxy, -NR'R', -O-C₁₋₆alkyle, -S-C₁₋₆alkyle, -U-C₃-₆cycloalkyl, -U-hétérocyclyle, -U-phényle, -U-naphthyle, -U-hétéroaryle, phénylalkyle, naphthylalkyle, hétérocycloalkyle et hétéroarylalkyle; dans lequel C₃₋₆cycloalkyle, hétérocyclyle, phényle, naphthyle, hétéroaryle et C₁₋₆alkyle sont optionnellement substitués par un, deux, trois, ou plus, substituants, chacun étant sélectionné dans R^{A1} ;
U est sélectionné dans le groupe constitué de -S-, -NR'-, -O- ou C₁₋₆alkyle ;
R^{C1}, R^{C2}, R^{C3} et R^{C4} sont sélectionnés indépendamment, pour chaque occurrence, dans le groupe constitué de hydrogène, halogène, hydroxyle, amine, amide, sulfonyle, sulfamide, thiol, C₁₋₆alkyle, haloC₁₋₆alkyle, carboxyle, cyano, nitro, alkoxy, C₃₋₆cycloalkyle, hétérocyclyle, phényle, naphthyle et hétéroaryle ;
ou deux substituants de R^{C3} ou deux substituants de R^{C4} peuvent être pris ensemble pour former un oxo, un imine ou un sulfanylidène ;
ou deux substituants de R^{C1}, deux substituants de R^{C2}, deux substituants de R^{C3} ou deux substituants de R^{C4} peuvent être pris ensemble avec l'atome ou les atomes auxquels ils sont attachés pour former un C₃₋₆cycloalkyle, un phényle, un naphtyle ou un hétérocycle saturé, partiellement saturé ou non saturé monocyclique à 4-6 éléments ou bi-cyclique à 10-12 éléments ayant un, deux ou trois hétéroatomes sélectionnés indépendamment dans le groupe constitué de N, O et S ;
ou un substituant de R^{C1} et un substituant de R^{C3}, ou un substituant de R^{C2} et un substituant de R^{C4} peuvent être pris ensemble avec les atomes auxquels ils sont attachés pour former un C₃₋₆cycloalkyle, un phényle, un naphtyle ou un hétérocycle saturé, partiellement saturé ou non saturé monocyclique à 4-6 éléments ou bi-cyclique à 10-12 éléments ayant un, deux ou trois hétéroatomes sélectionnés indépendamment dans le groupe constitué de N, O et S ;
R¹ est sélectionné indépendamment, pour chaque occurrence, dans le groupe constitué de halogène, hydroxyle, amine, amide, sulfonyle, sulfamide, thiol, C₁₋₆alkyle, haloC₁₋₆alkyle, carboxyle, -C(O)O-C₁₋₆alkyle, -OC(O)O-C₁₋₆alkyle, -OC(O)NR'R', -N(R')C(O)NR'R', -N(R')C(O)O-C₁₋₆alkyle, cyano, nitro, alkoxy, C₃₋₆cycloalkyle, hétérocyclyle, hétéroaryle et phényle ;
R^{A1} est sélectionné indépendamment, pour chaque occurrence, dans le groupe constitué de halogène, hydroxyle, amine, amide, sulfonyle, sulfamide, thiol, C₁₋₆alkyle, haloC₁₋₆alkyle, carboxyle, cyano, nitro, alkoxy, -C(O)O-C₁₋₆alkyle, -OC(O)O-C₁₋₆alkyle, -OC(O)NR'R', -N(R')C(O)NR'R', -N(R')C(O)O-C₁₋₆alkyle, oxo, C₃₋₆cycloalkyle, hétérocyclyle, hétéroaryle et phényle ;
R^{N1} et R' sont sélectionnés indépendamment, pour chaque occurrence, dans le groupe constitué de hydrogène et C₁₋₆alkyle ; dans lequel C₁₋₆alkyle est optionnellement substitué par un, deux, trois, ou plus, substituants sélectionnés indépendamment, pour chaque occurrence, dans R^{A1} ;
ou deux substituants de R^{N1} ou deux substituants de R' peuvent être pris ensemble avec l'azote auquel ils sont attachés pour former un hétérocycle saturé, partiellement saturé ou non saturé monocyclique à 4-7 éléments ou bi-cyclique à 10-15 éléments ayant un, deux ou trois hétéroatomes sélectionnés indépendamment dans le groupe constitué de N, O et S ;
ou un substituant de R^{N1} et un substituant de R^{C4} peuvent être pris ensemble avec les atomes auxquels ils sont attachés pour former un hétérocycle saturé, partiellement saturé ou non saturé monocyclique à 4-7 éléments ou bi-cyclique à 10-15 éléments ayant un, deux ou trois hétéroatomes sélectionnés indépendamment dans le groupe constitué de N, O et S ;
m est sélectionné dans le groupe constitué de 0, 1, 2 et 3; ou
un sel ou un stéréoisomère de celui-ci acceptable du point de vue pharmaceutique.

2. Composé hétérocyclique selon la revendication 1, dans lequel A¹ est C₃₋₆cycloalkyle.

3. Composé hétérocyclique selon la revendication 2, dans lequel A¹ est sélectionné dans le groupe constitué de cyclohexyle, cyclohexényle, cyclopentyle, cyclopentényle, cyclobutyle, cyclopropyle et toute variante pontée, spiralée et ou fusionnée de ceux-ci.

4. Composé hétérocyclique selon l'une quelconque des revendications 1 à 3, dans lequel 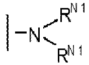 est sélectionné dans le groupe constitué de : 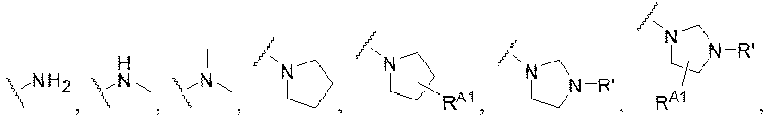 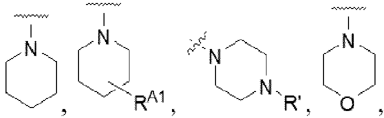 et

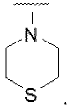

5. Composé hétérocyclique sélectionné dans le groupe constitué de : 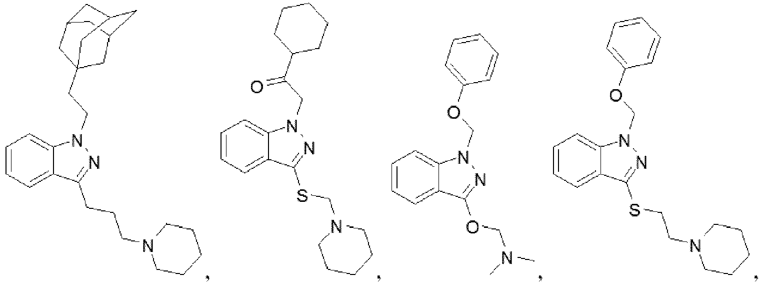 et 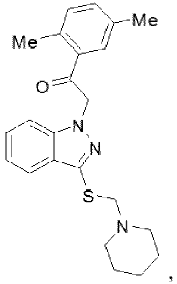 ou un sel de celui-ci acceptable du point de vue pharmaceutique.

6. Composé hétérocyclique selon la revendication 5, dans lequel le composé hétérocyclique est : 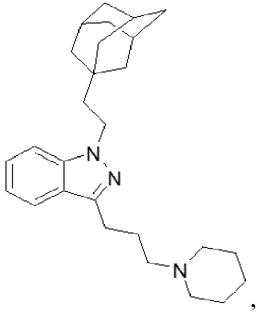 ou un sel de celui-ci acceptable du point de vue pharmaceutique.

7. Composé représenté par : 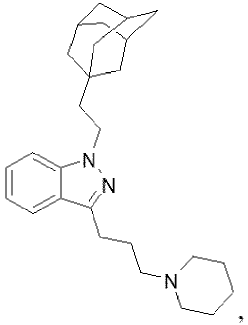 ou un sel de celui-ci acceptable du point de vue pharmaceutique, pour utilisation dans le traitement d'une maladie ou d'un dérangement inflammatoire immun.

8. Composé pour une utilisation selon la revendication 7, dans lequel le dérangement inflammatoire immun est sélectionné dans le groupe constitué de la maladie de Crohn, la rectocolite hémorragique, un dérangement rhumatismal, le psoriasis et une allergie.

9. Composé pour une utilisation selon la revendication 8, dans lequel l'allergie est une dermatite de contact ou une dermatite atopique.

10. Composition pharmaceutique comprenant un composé selon l'une quelconque des revendications 1 à 6 et un adjuvant ou un excipient acceptable du point de vue pharmaceutique.
